# EUROPEAN PATENT SPECIFICATION

(11) **EP 0 567 133 B1**
(45) Date of publication and mention of the grant of the patent: **03.11.1999**
(21) Application number: 93106574.2
(22) Date of filing: 22.04.1993
(51) Int. Cl.: C07D 401/12, C07D 405/14, C07D 401/14, C07D 409/14, C07D 413/14, C07D 401/06, A01N 43/54

(54) **Picolinic acid derivatives and herbicidal compositions**
Picolinsäurederivate und herbizide Zusammensetzungen
Dérivés de l'acide picolinique et compositions herbicides

(30) Priority: 23.04.1992 JP 12937692
(43) Date of publication of application: 27.10.1993
(73) Proprietor: KUMIAI CHEMICAL INDUSTRY CO., LTD., Taitoh-ku Tokyo 110-91 (JP); IHARA CHEMICAL INDUSTRY CO., LTD., Taitoh-ku, Tokyo 110-0008 (JP)
(72) Inventor: Takabe, Fumiaki, Fukude-cho, Iwata-gun, Shizuoka-ken (JP); Saito, Yoshihiro, Fukude-cho, Iwata-gun, Shizuoka-ken (JP); Tamaru, Masatoshi, Fukude-cho, Iwata-gun, Shizuoka-ken (JP); Tachikawa, Shigehiko, Ogasa-gun, Shizuoka-ken (JP); Hanai, Ryo, Ogasa-gun, Shizuoka-ken (JP)
(74) Representative: Wächtershäuser, Günter, Prof. Dr.

(56) References cited:
- EP-A- 0 249 707
- EP-A- 0 249 708
- EP-A- 0 346 789
- EP-A- 0 360 163
- EP-A- 0 461 079
- EP-A- 0 472 925
- EP-A- 0 507 962
- EP-A- 0 521 407
- WO-A-91/10653

## Description

### BACKGROUND OF THE INVENTION

### FIELD OF THE INVENTION

The present invention relates to a picolinic acid derivative and a salt thereof, and a herbicidal composition containing the same as an active ingredient, which is applicable to a paddy field, an upland field and a non-agricultural land.

### DISCUSSION OF BACKGROUND

EP-A-0 521 407, EP-A-0 472 925 and EP-A-0 249 707 disclose picolinic acid derivatives.

EP-A-0 346 789 and EP-A-0 249 708 disclose benzoic acid derivatives.

As a herbicide having a structure similar to the compound of the present invention, a 3-(4,6-dimethoxypyrimidin-2-yl)oxypicolinic acid derivative has been known (Japanese Unexamined Patent Publication No. 121973/1990). This publication discloses a picolinic acid type (2-pyridine-carboxylic acid) structure and an isonicotinic acid type (4-pyridine-carboxylic acid) structure as a compound having a pyridine ring. However, this publication does not disclose a compound having a substituent on the pyridine ring, like the compound of the present invention. Accordingly, there is no specific disclosure or suggestion of the herbicidal effects of such a compound.

Further, it is also known that a 2-sulfonaminopyrimidine derivative has herbicidal activities (Japanese Unexamined Patent publication No. 149567/1990). However, in this case, a large dose is required to control various weeds simultaneously, and its herbicidal effects are not yet satisfactory.

Furthermore, a similar patent publication (WO-9207846-A1) discloses herbicidal effects of an amino group derivative at the 6-position of picolinic acid. However, there is no specific description about the safety of the compound to crop plants, and the herbicidal effects are not satisfactory particularly to control weeds in an agricultural field.

In recent years, a number of herbicides have been developed and practically used, and they have contributed to the saving of energy for the agricultural operations and to the improvement of the production efficiency. However, in their practical use, such herbicides have various problems with respect to the herbicidal effects and the safety to the crop plants. For example, perennial weeds such as Johnsongrass are widely distributed throughout agricultural fields in the world and regarded as weeds which are very difficult to control. Under the circumstances, it is desired to develop an improved herbicide.

### SUMMARY OF THE INVENTION

The present inventors have conducted extensive research on picolinic acid derivatives with an aim to solve the above-mentioned problems and as a result, have found that a picolinic acid derivative substituted by a pyrimidinyloxy group exhibits excellent herbicidal effects against annual and perennial gramineous and broadleaf weeds, and at the same time, it is highly safe to crop plants such as corn. It has been also found that the picolinic acid derivative exhibits excellent herbicidal effects at a low dose especially against annual gramineous weeds, and at the same time, adequate herbicidal effects can be obtained also against perennial weeds such as Johnsongrass. The present invention has been accomplished on the basis of these discoveries.

Thus, the present invention provides a herbicide containing as an active ingredient a picolinic acid derivative of the formula: 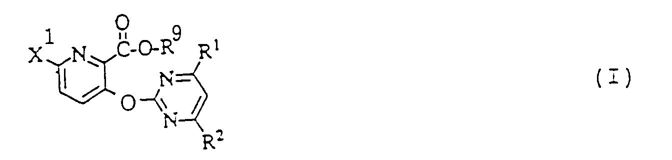 wherein each of R¹ and R² is a C₁₋₅-alkoxy group, X¹ is a group of the formula 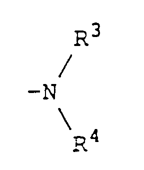 wherein R³ is a hydrogen atom, a C₁₋₅ alkyl group, a halo C₁₋₅ alkyl group, a C₁₋₅ alkoxy C₁₋₅ alkyl group, a C₂₋₆ alkenyl group, a C₂₋₆ alkynyl group, a C₁₋₅ alkylcarbonyl group, a benzyloxycarbonyl group or a phenylsulfonyl group, and R⁴ is a halo C₁₋₅ alkyl group, a C₁₋₅ alkoxy C₁₋₅ alkyl group, a C₁₋₅ alkoxy C₁₋₅ alkyloxy C₁₋₅ alkyl group, a C₁₋₅ alkoxycarbonyl C₁₋₅ alkyl group, a benzyloxy C₁₋₅ alkyl group, a C₁₋₅ alkyldioxy C₁₋₅ alkyl group, a phenoxy C₁₋₅ alkyl group, a di C₁₋₅ alkylamino C₁₋₅ alkyl group, a cyano C₁₋₅ alkyl group, a C₁₋₅ alkoxy group, a C₃₋₆ cycloalkyl group, a C₂₋₆ alkenyl group (which may be substituted at one or more positions by a halogen atom or a cyano group), a C₂₋₆ alkynyl group, a phenyl C₁₋₅ alkyl group (which may be the same or different, and substituted at one or more positions by a halogen atom, a C₁₋₅ alkyl group, a C₁₋₅ alkoxy group, a nitro group or a cyano group), a cyclo C₁₋₅ alkylcarbonyl group, a halo C₁₋₅ alkylcarbonyl group, a C₁₋₅ alkoxy C₁₋₅ alkylcarbonyl group, a cyano C₁₋₅ alkylcarbonyl group, a phenoxy C₁₋₅ alkylcarbonyl group, a phenyl C₁₋₅ alkylcarbonyl group, a halophenyl C₁₋₅ alkyl carbonyl group, a benzoyl group (which is substituted by a C₁₋₅ alkyl group, a halo C₁₋₅ alkyl group, a halogen atom, a C₁₋₅ alkoxy group, a cyano group or a nitro group), a furylcarbonyl group, a pyridylcarbonyl group, a pyrrolylcarbonyl group, a thienylcarbonyl group, a C₂₋₆ alkenylcarbonyl group, a phenyl C₂₋₆ alkenylcarbonyl group, a hydroxycarbonyl C₂₋₆ alkenylcarbonyl group, a C₁₋₅ alkoxycarbonyl C₁₋₅ alkylcarbonyl group, a C₁₋₅ alkoxy C₁₋₅ alkoxycarbonyl group, a mono C₁₋₅ alkylaminocarbonyl group, a di C₁₋₅ alkylaminocarbonyl group, a phenylaminocarbonyl group, a benzylaminocarbonyl group, a halobenzylaminocarbonyl group, a C₁₋₅ alkoxycarbonyl group, a halo C₁₋₅ alkoxycarbonyl group, a benzyloxycarbonyl group, a C₂₋₆ alkenyloxycarbonyl group, a C₂₋₆ alkynyloxycarbonyl group, a C₁₋₅ alkylsulfonyl group, a halo C₁₋₅ alkylsulfonyl group, a benzylsulfonyl group which may be substituted by a C₁₋₅ alkoxycarbonyl group, a phenylsulfonyl group which may be substituted by a C₁₋₅ alkoxycarbonyl group, a halophenylsulfonyl group, a C₁₋₅ alkylthiocarbonyl group, a halo C₁₋₅ alkylthiocarbonyl group, a benzylthiocarbonyl group, a halobenzylthiocarbonyl group, a C₂₋₆ alkenylthiocarbonyl group, a C₂₋₆ alkynylthiocarbonyl group which may be substituted by a cyano group, a C₁₋₅ alkylamino(thiocarbonyl) group, a phenylamino(thiocarbonyl) group, a di C₁₋₅ alkylamino(thiocarbonyl) group, a benxyloxy group or a group of the formula 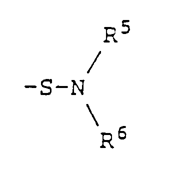 wherein each of R⁵ and R⁶ which may be the same or different, is a hydrogen atom, a C₁₋₅ alkyl group or a C₁₋₅ alkoxycarbonyl group, or R⁵ and R⁶ form together with the adjacent nitrogen atom a morpholino group, or R³ and R⁴ form together with the adjacent nitrogen atom, an azido group, an isothiocyanate group₁ a phthalimide group, a maleimide group, a succinimido group, a pyrrolidinyl group, a piperidino group, a pyrrolyl group, a morpholino group, a group of the formula 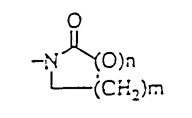 wherein n is 0 or 1, and m is 1 or 2, or a group of the formula 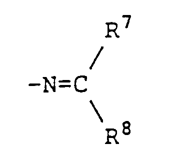 wherein each of R⁷ and R⁸ which may be the same or different, is a hydrogen atom, a C₁₋₅ alkyl group, a di C₁₋₅ alkylamino group, an amino group, a C₁₋₅ alkylthio group, a phenyl group or a benzyl group, and R⁹ is a hydrogen atom, a C₁₋₅ alkyl group, a C₂₋₆ alkenyl group, a benzyl group, an alkali metal atom, an alkaline earth metal atom or an organic amine cation; or a salt thereof. Further, the present invention provides a picolinic acid derivative of the formula: 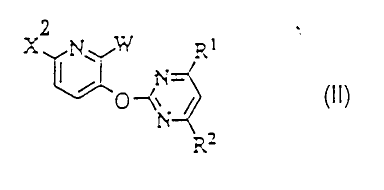 wherein each of R¹ and R² is a C₁₋₅ alkoxy group, X² is an amino group, a C₁₋₅ alkylamino group or a di C₁₋₅ alkylamino group, and W is COOR¹⁰, COSR¹¹ or 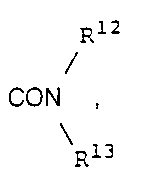 wherein R¹⁰ is a phenyl group (which may be substituted by a halogen atom or a methyl group), a benzyloxy C₁₋₅ alkyl group, a C₃₋₁₂ alkylideneamino group, a C₃₋₈ cycloalkylideneamino group or a di C₁₋₅ alkylamino group, R¹¹ is a C₁₋₅ alkyl group or a phenyl group, and each of R¹² and R¹³ which may be the same or different, is a hydrogen atom, a C₁₋₅ alkylsulfonyl group, a C₁₋₅ alkoxy C₁₋₅ alkyl group, a C₁₋₅ alkyl group, a benzyloxy group or a C₁₋₅ alkoxy group, or R¹² and R¹³ form together with the adjacent nitrogen atom, an imidazolyl group; or a salt thereof.

The present invention also provides a herbicidal composition comprising a herbicidally effective amount of a picolinic acid derivative of the formula (I) or (II) or a salt thereof as defined above and an agricultural adjuvant.

### DETAILED DESCRIPTION OF THE PREFERRED EMBODIMENTS

Now, preferred examples of the substituents will be given. The alkyl and alkoxy groups may, for example, be C₁₋₅ linear or branched alkyl and alkoxy groups. The alkenyl and alkynyl groups may, for example, be C₂₋₆ alkenyl and alkynyl groups. The cycloalkyl group may, for example, be a C₃₋₆ cycloalkyl group. The alkylideneamino group may, for example, be a C₃₋₁₂ alkylideneamino group. The cycloalkylideneamino group may, for example, be a C₃₋₈ cycloalkylideneamino group. The acyl group may, for example, be a C₂₋₈ acyl group. The halogen atom may, for example, be a chlorine atom, a fluorine atom, a bromine atom or an iodine atom.

Now, specific compounds of the present invention will be given in Tables 1 to 3. The compound Nos. will be referred to in the subsequent description. In the Tables, D. P. means the decomposition point.

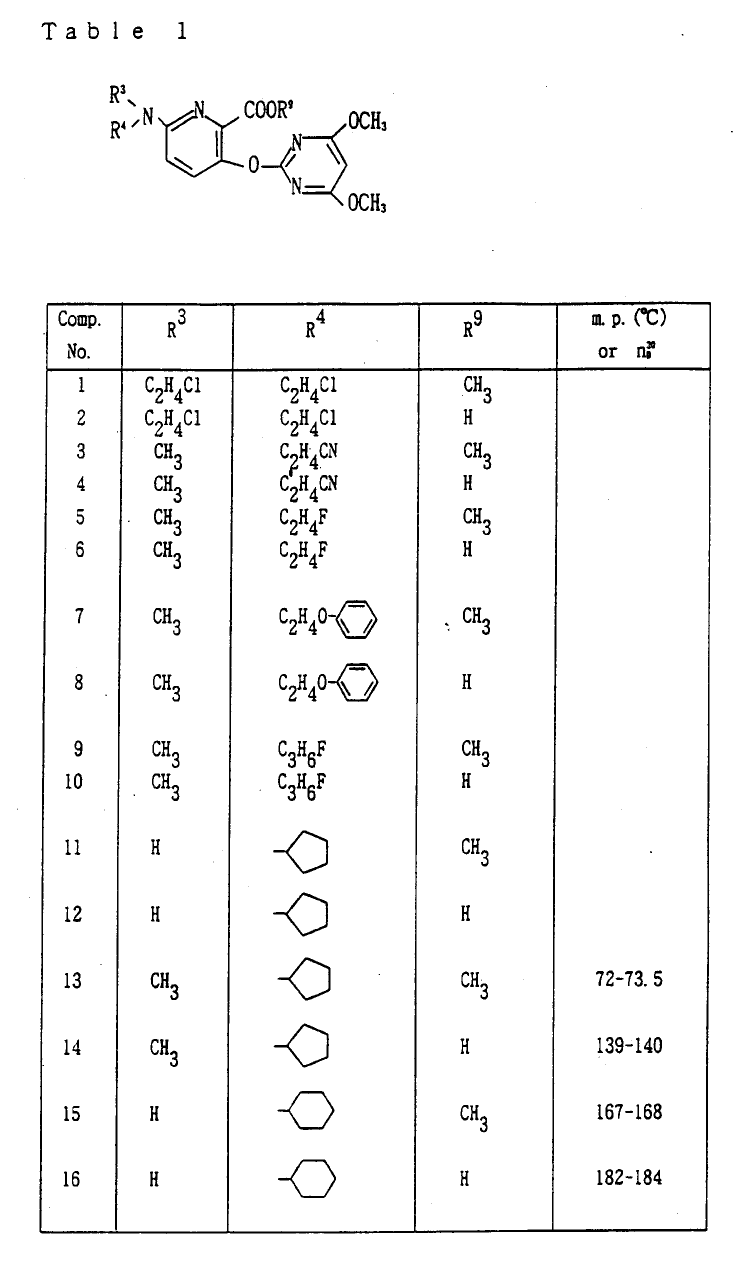

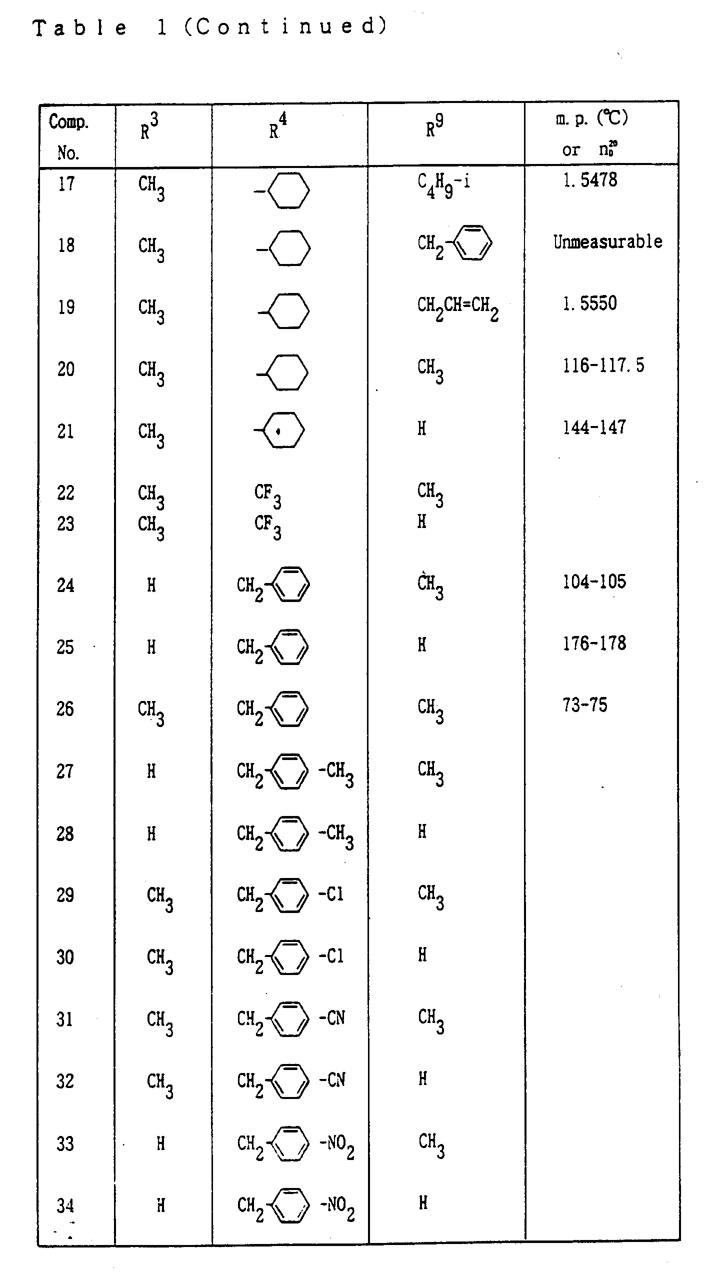

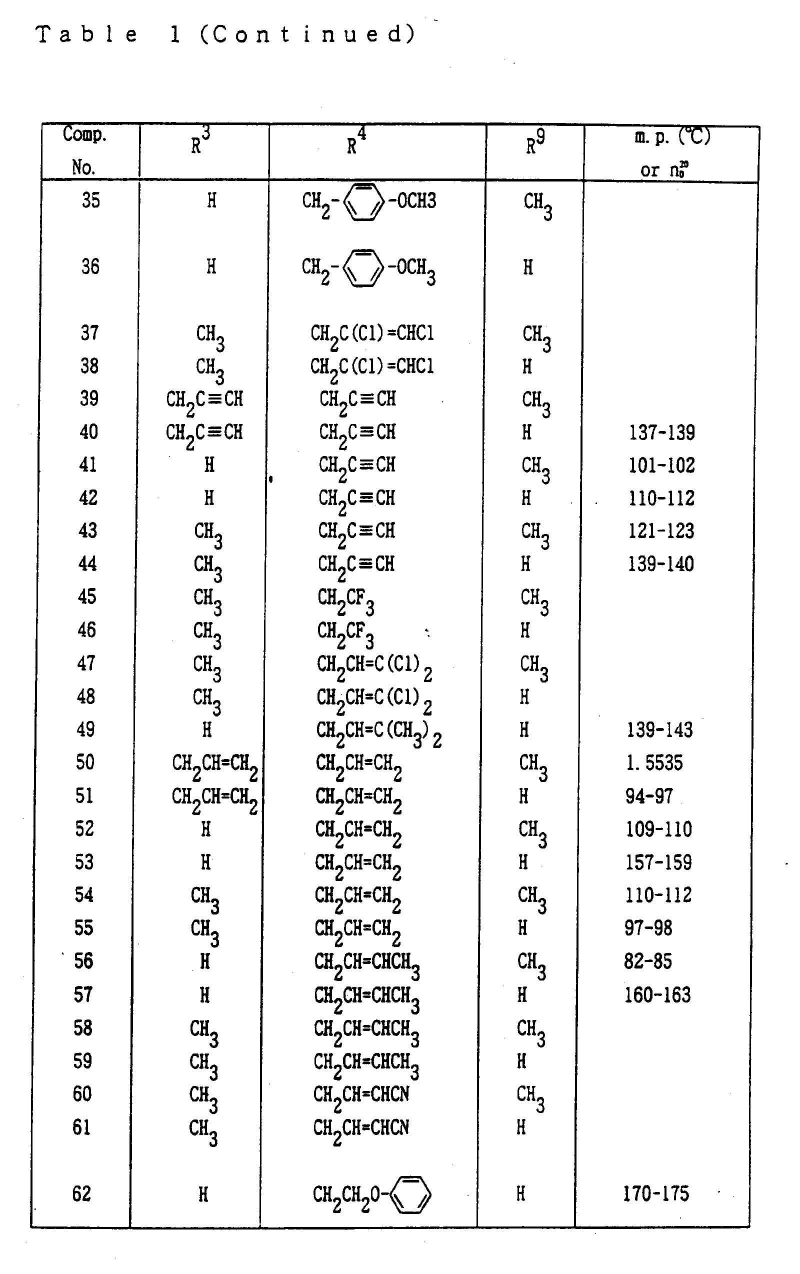

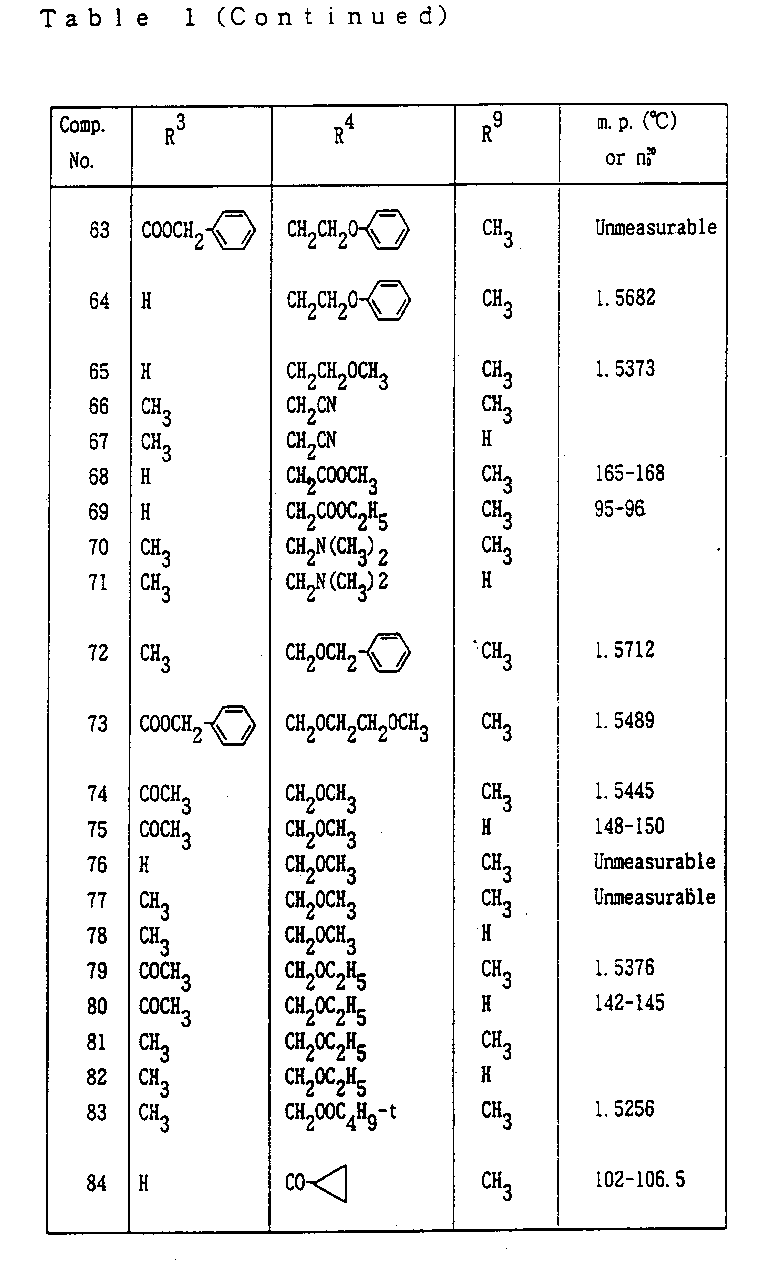

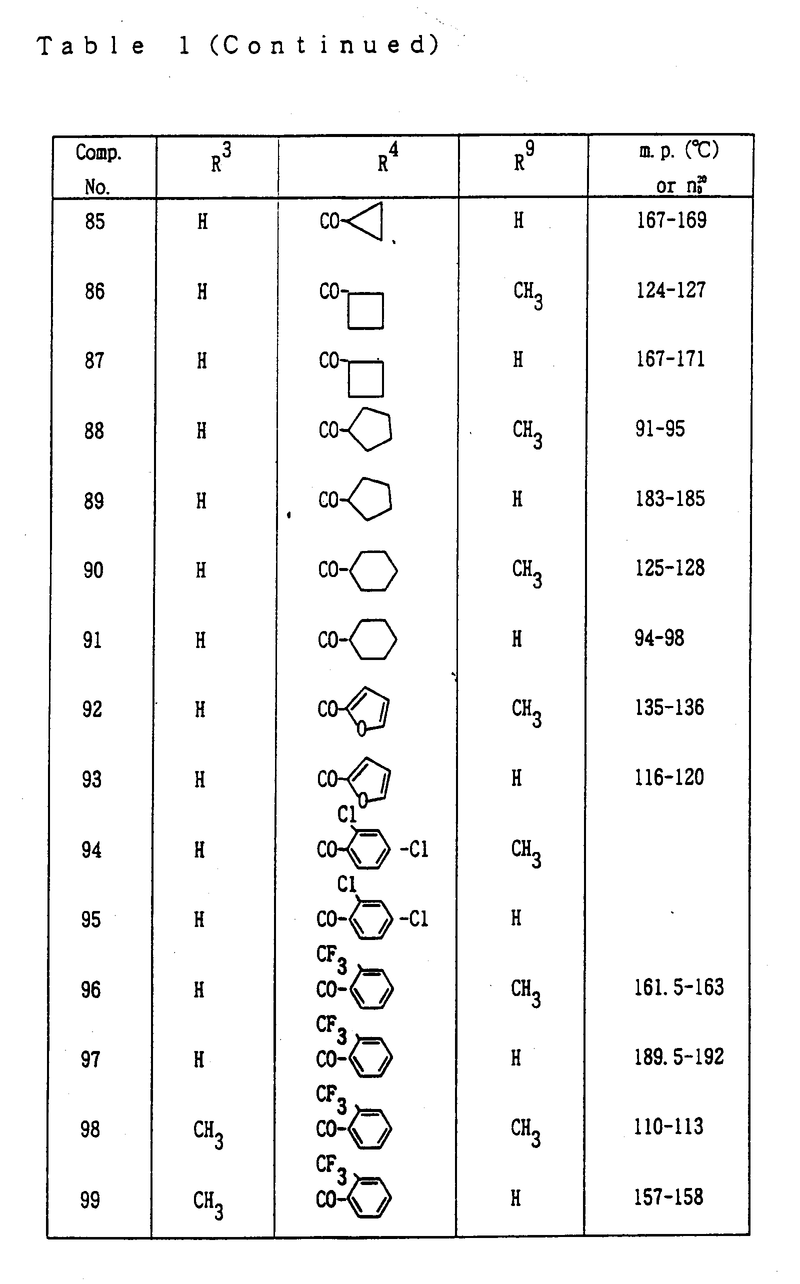

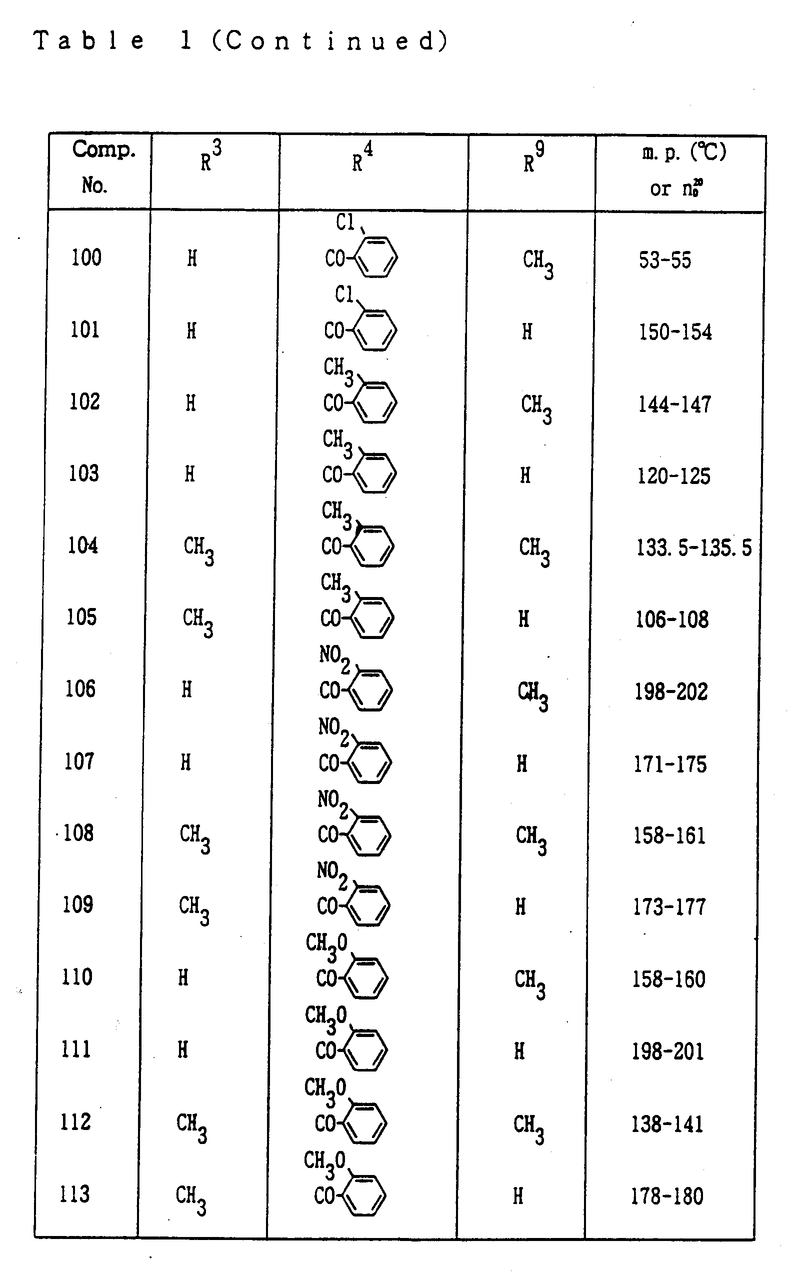

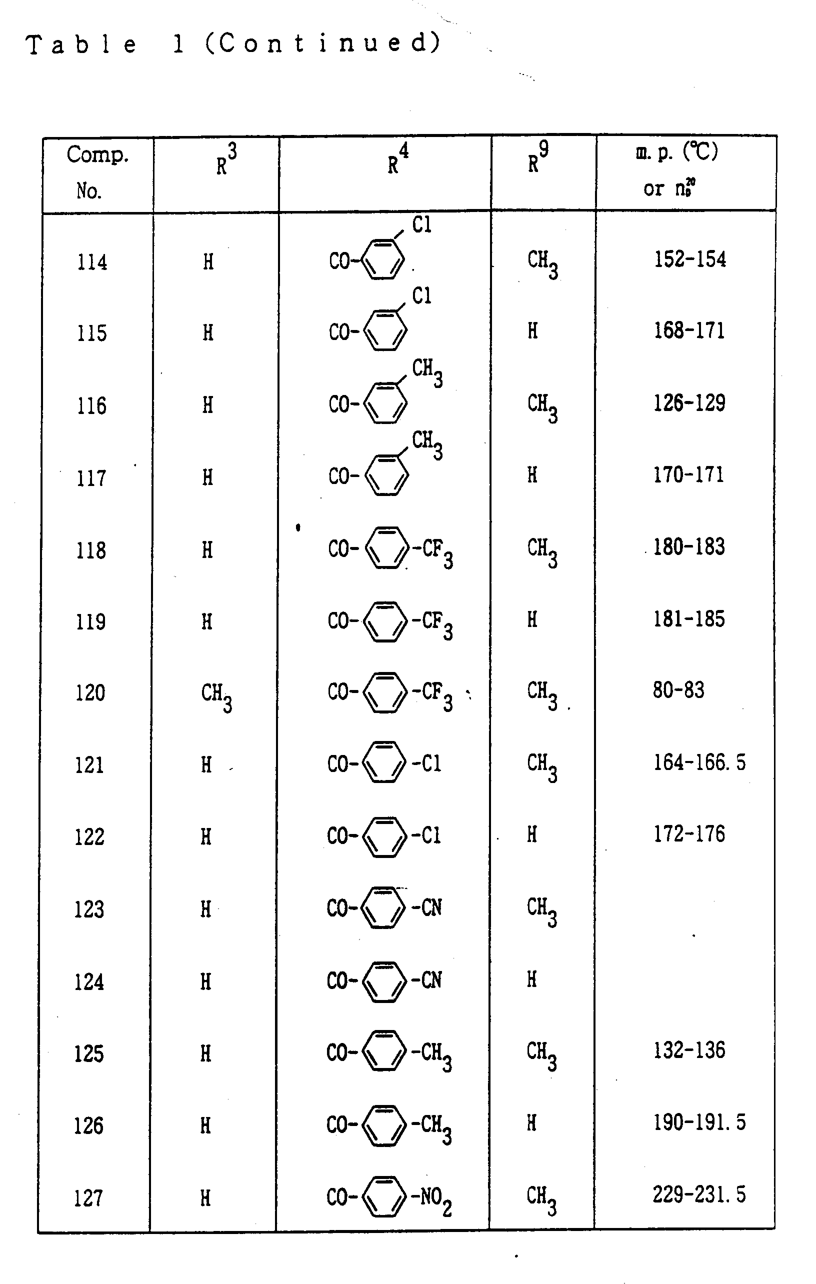

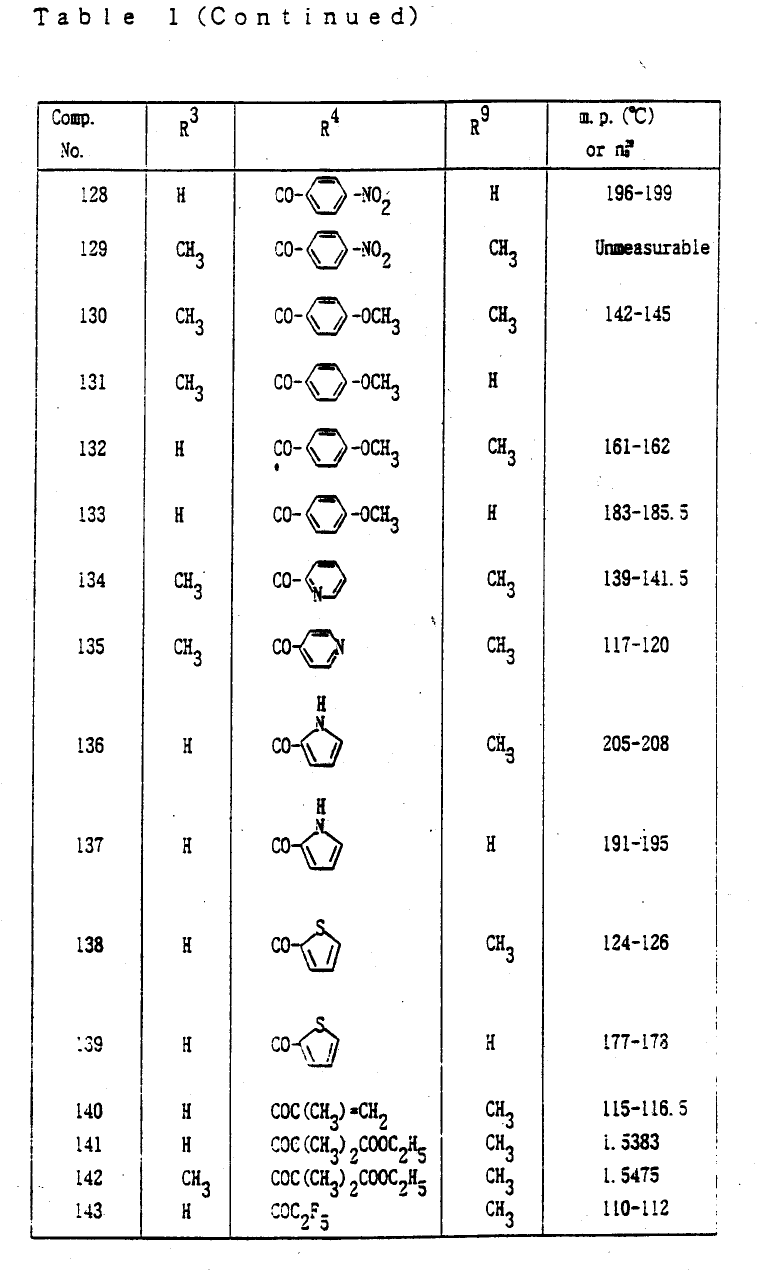

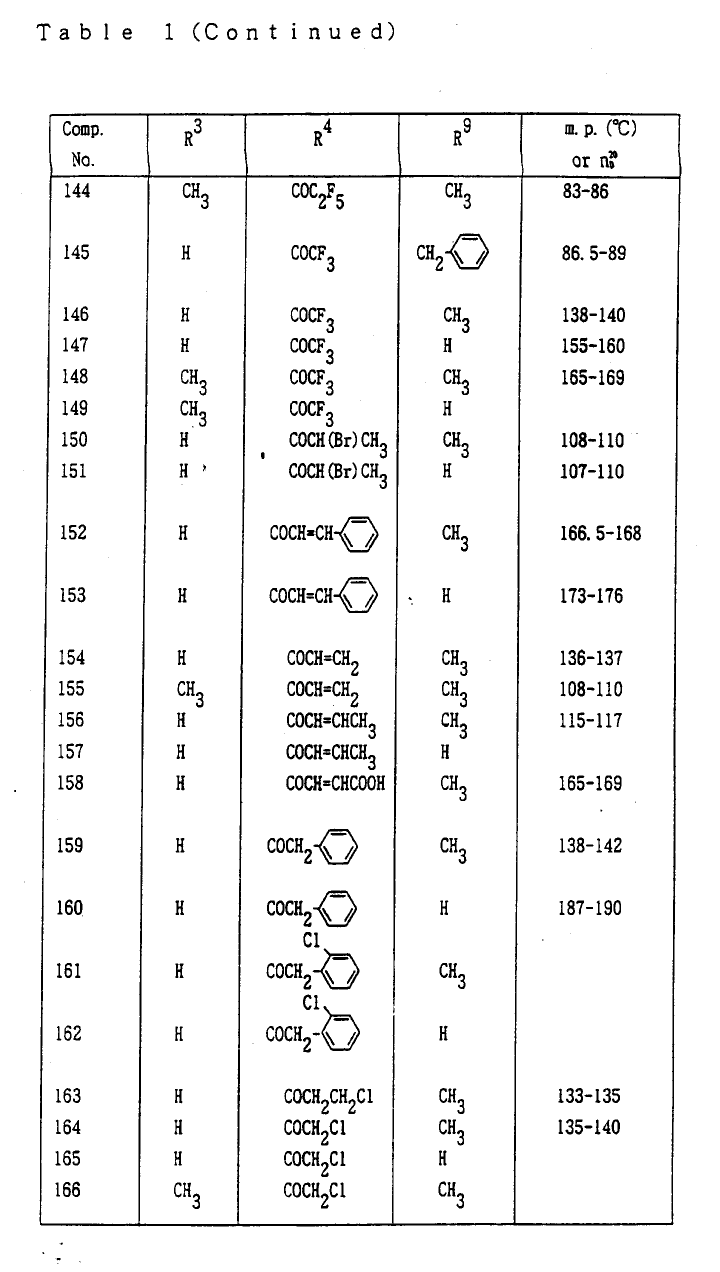

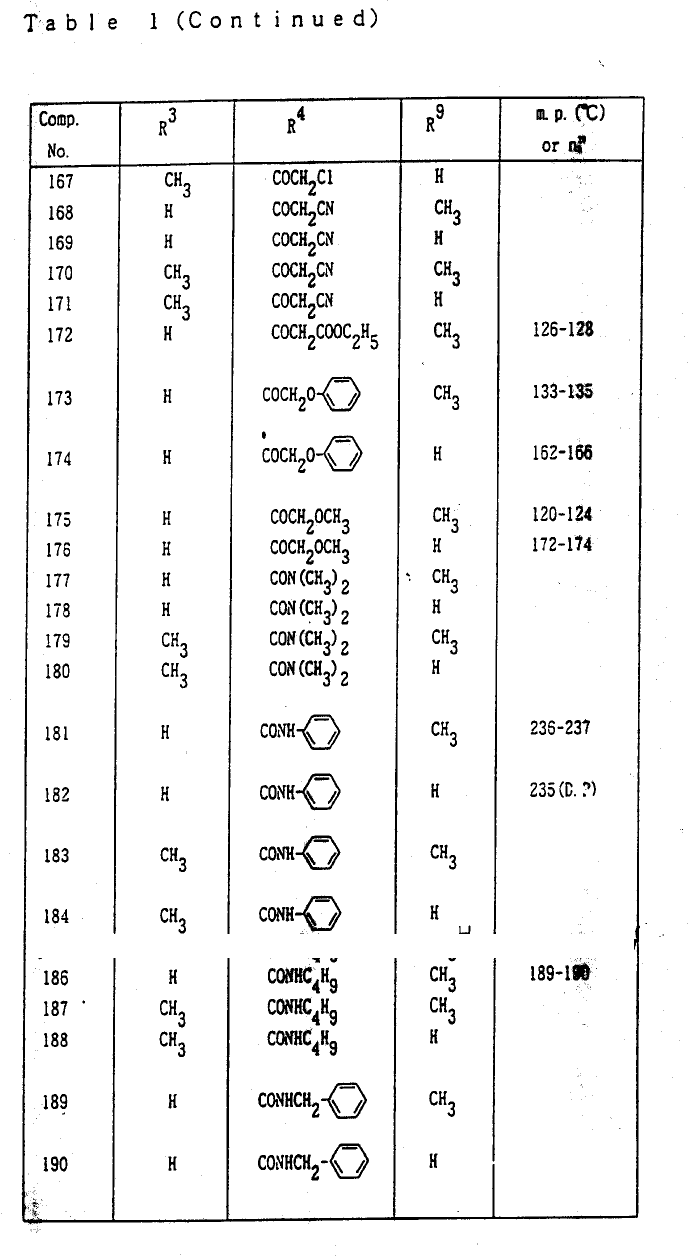

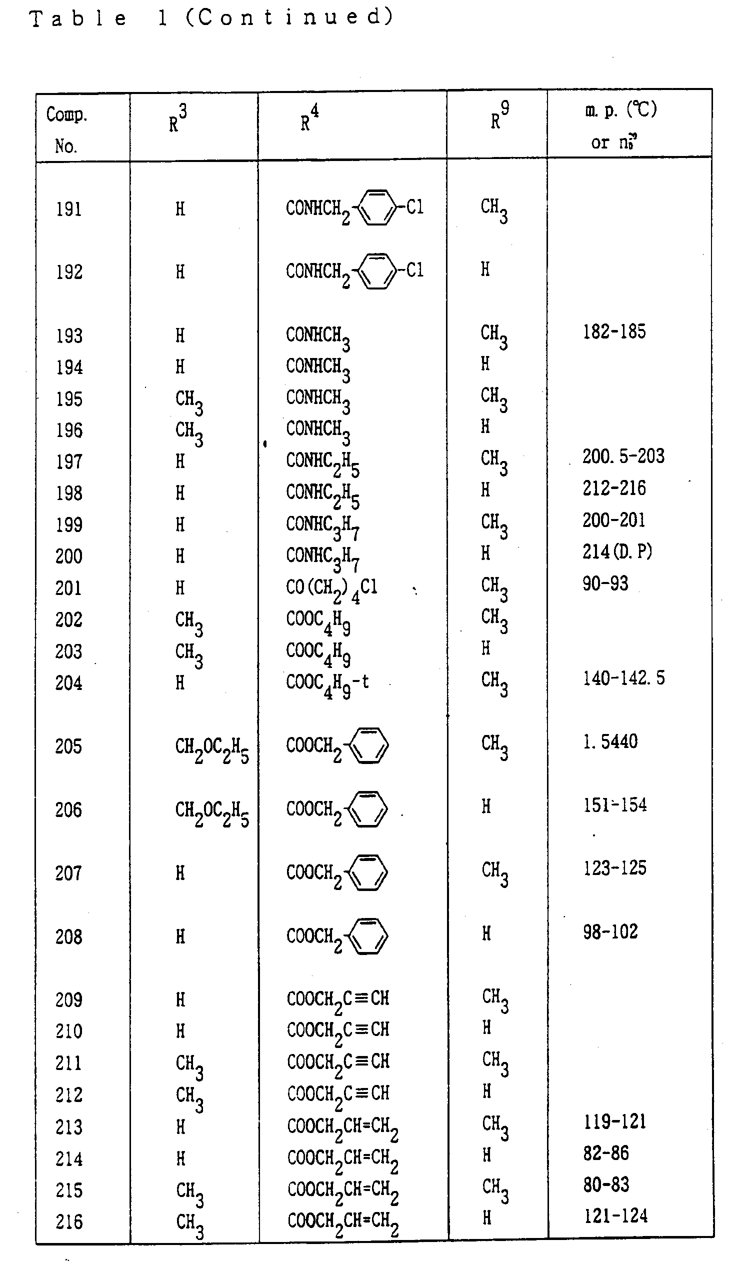

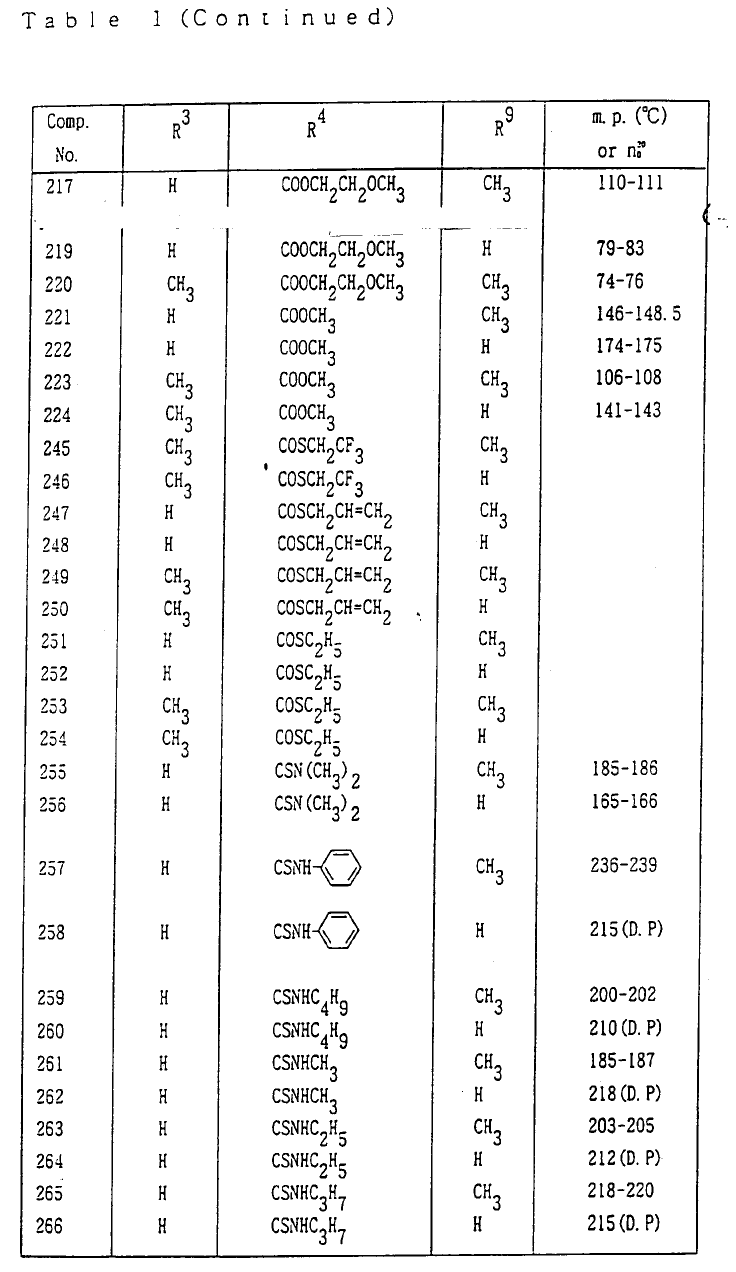

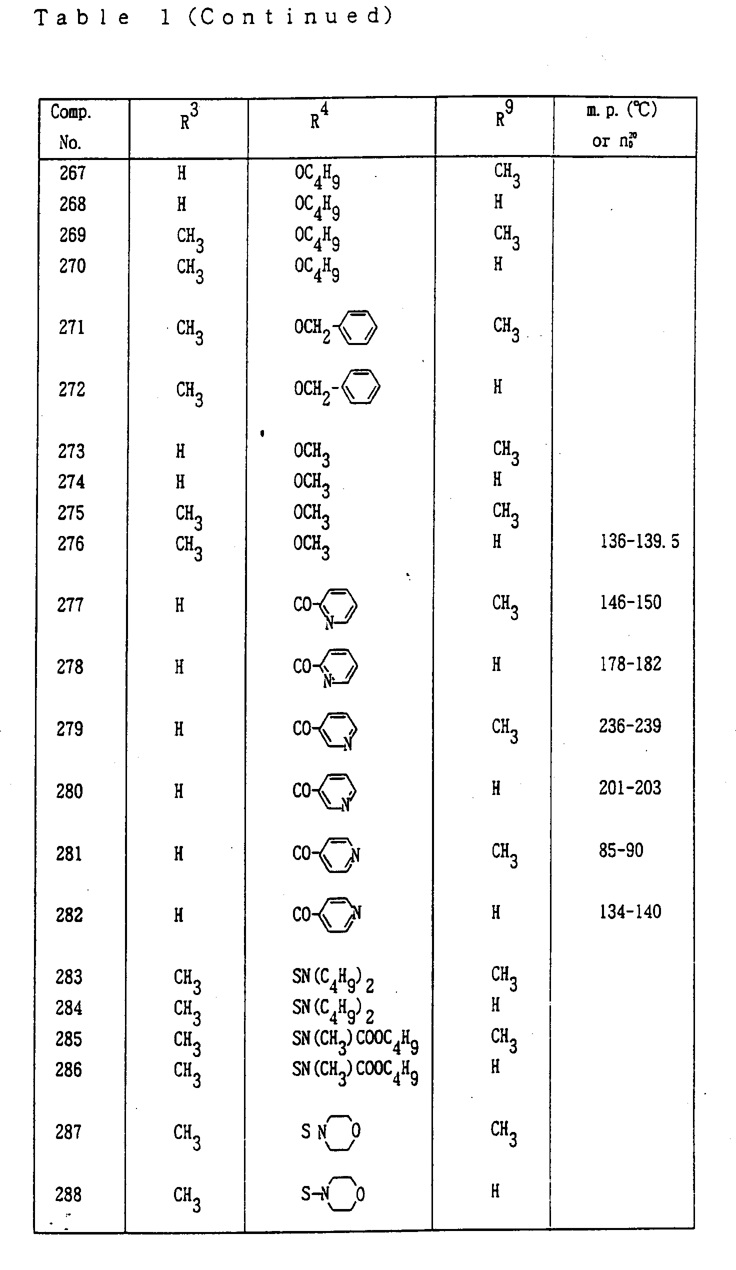

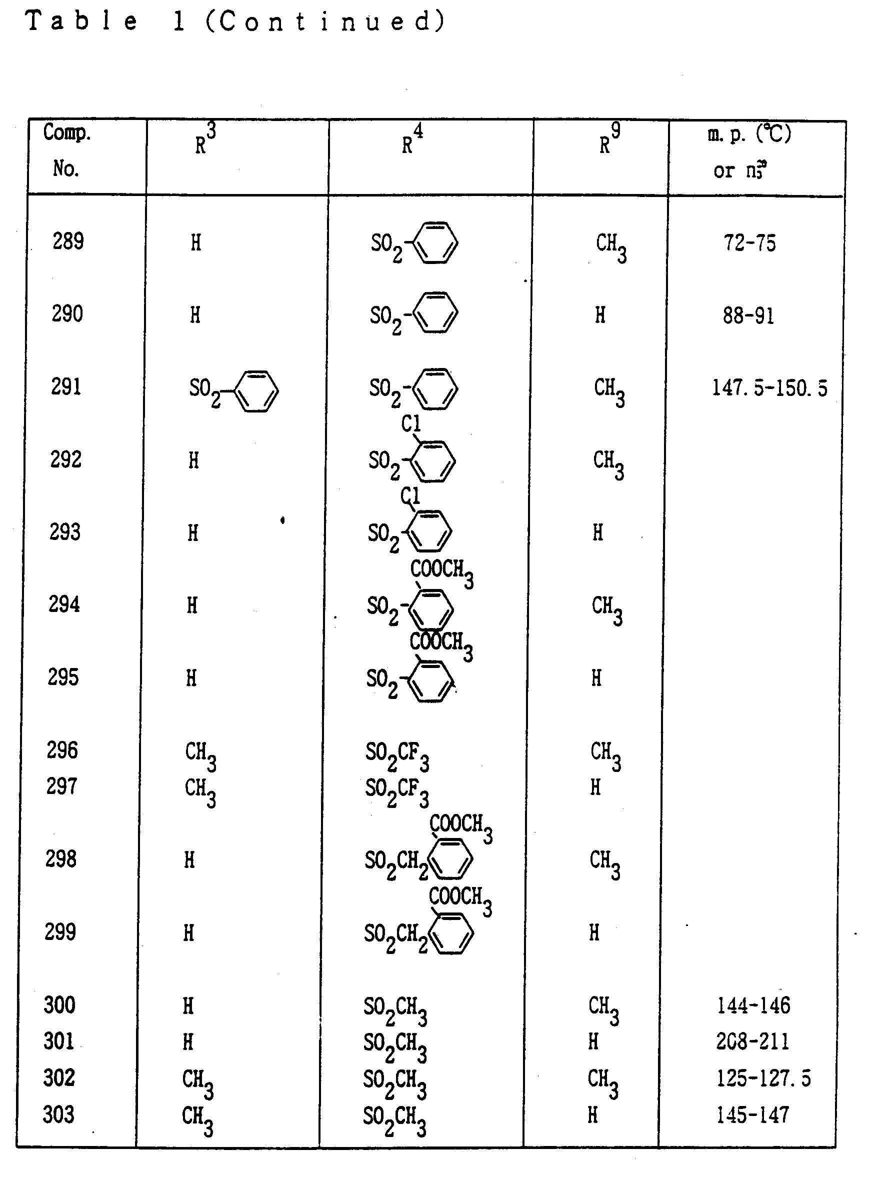

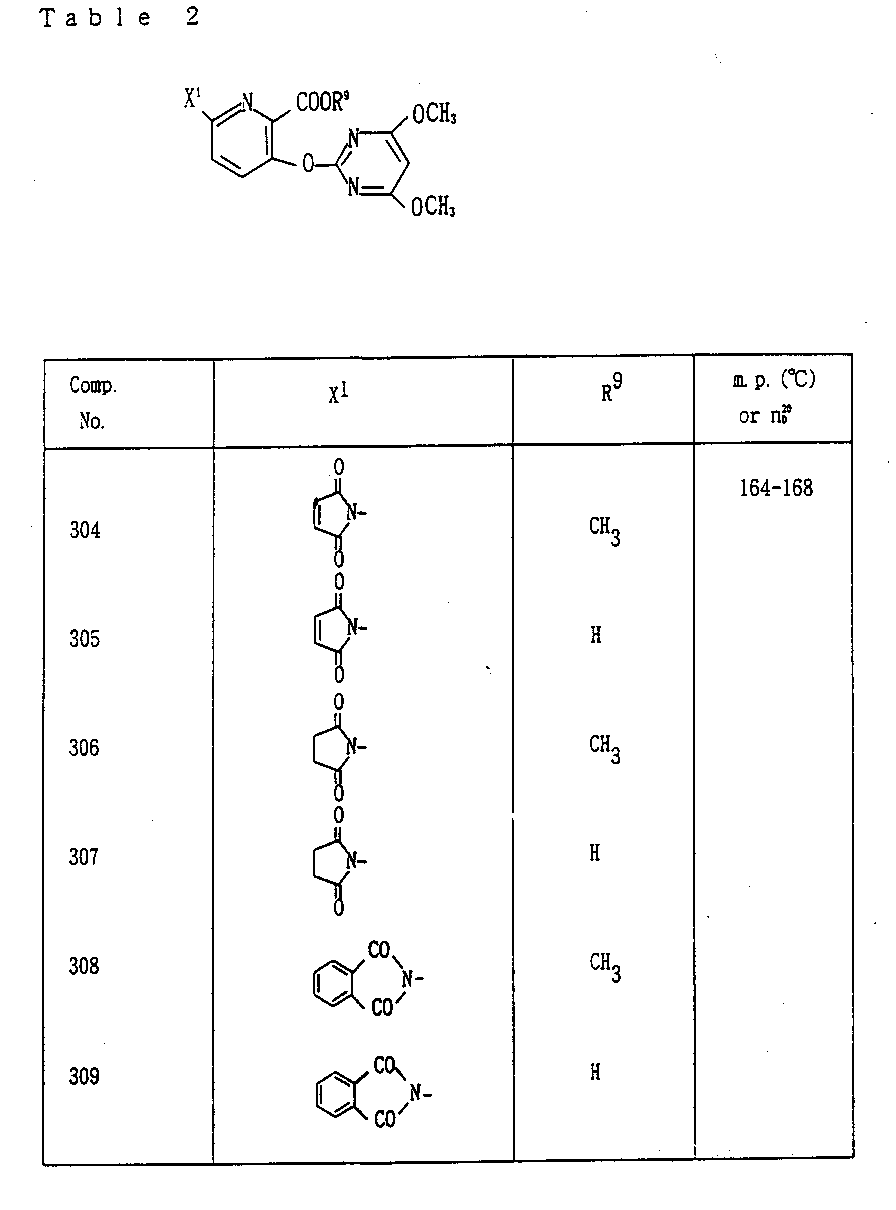

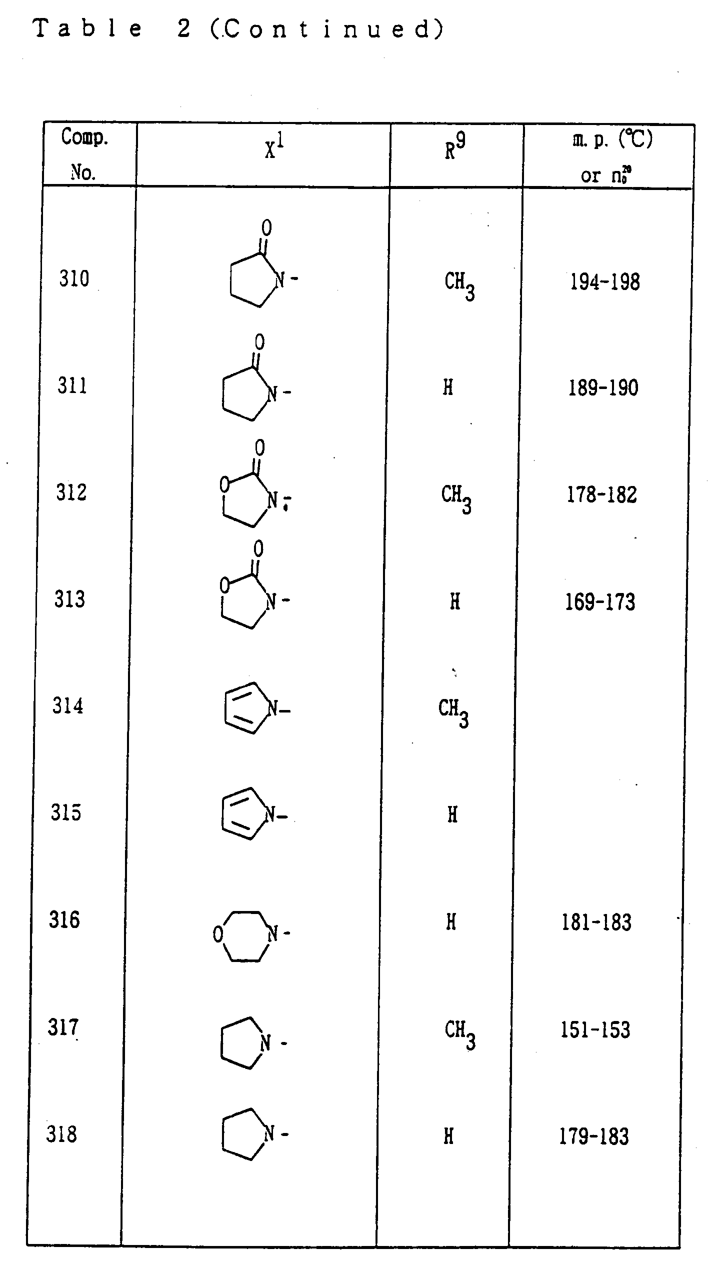

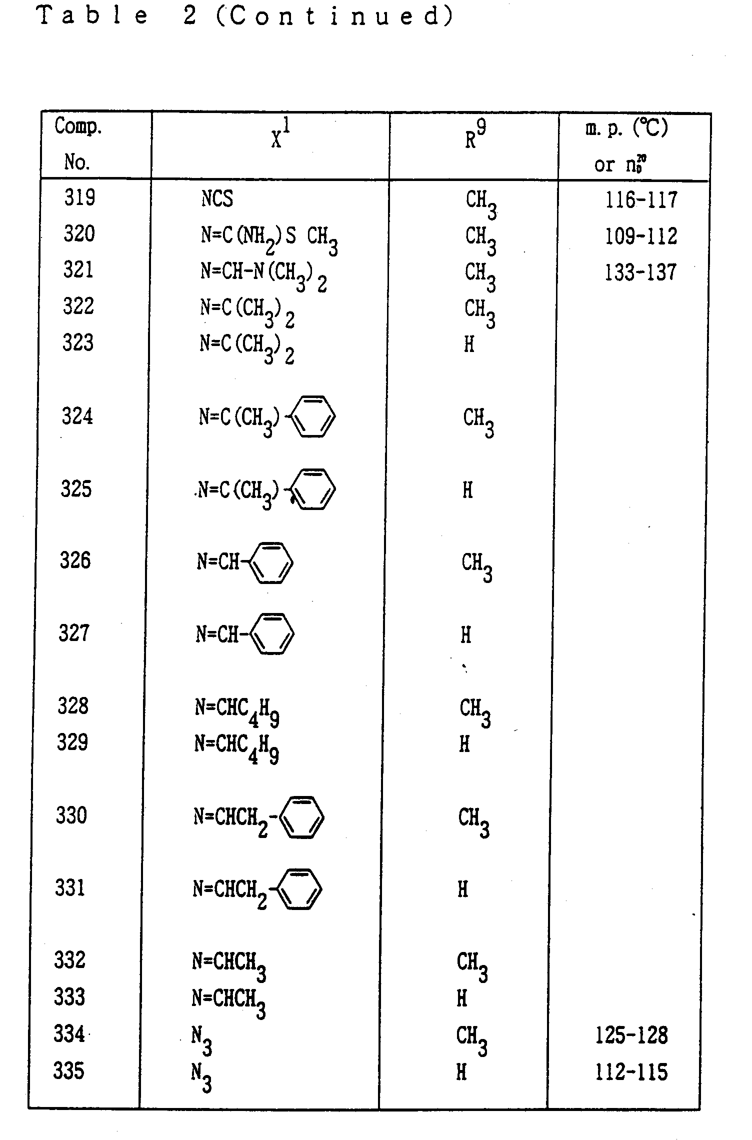

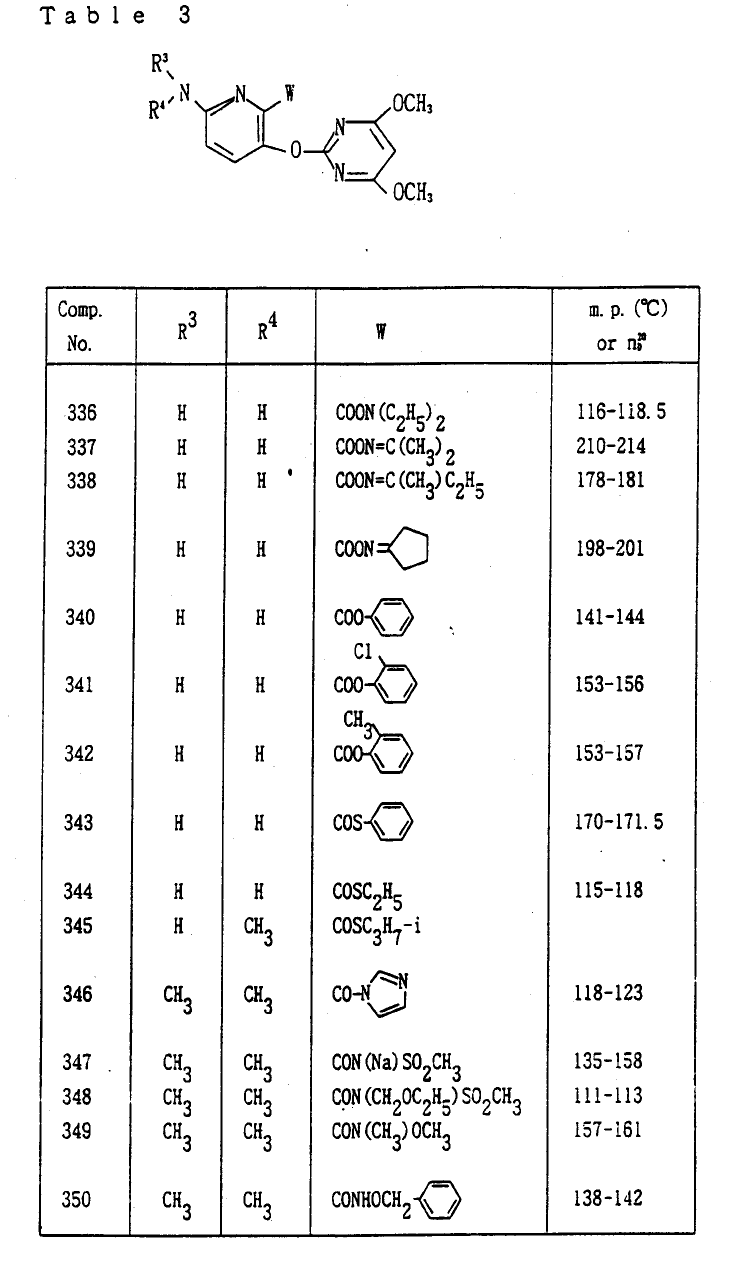

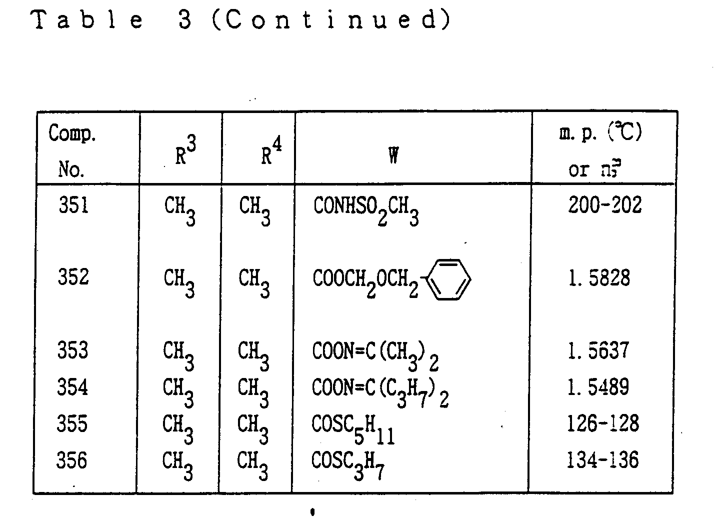

As a method for producing compounds of the present invention, the following processes A to K may, for example, be mentioned;

### Process A

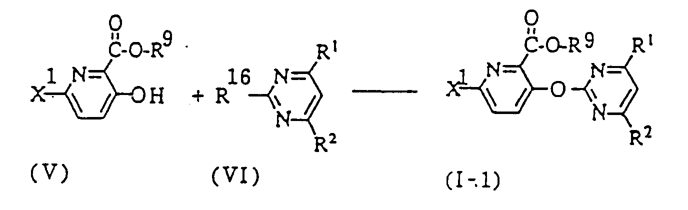

In the above formulas, R¹, R², R⁹ and X¹ are as defined above, and R¹⁶ is a halogen atom, an alkylsulfonyl group or a benzylsulfonyl group.

Namely, the compound of the formula (I-1) of the present invention can be produced by reacting the compound of the formula (V) and the pyrimidine derivative of the formula (VI) in the presence of a base, preferably in an inert solvent, within a temperature range of from 0°C to the boiling point of the solvent for from a few minutes to dozens of hours.

The solvent may be a hydrocarbon solvent such as benzene or toluene, a halogenated hydrocarbon solvent such as dichloromethane or chloroform, an ether solvent such as tetrahydrofuran or 1,4-dioxane, a ketone solvent such as acetone or methyl ethyl ketone (MEK), an ester solvent such as methyl acetate or ethyl acetate, an aprotic polar solvent such as N,N-dimethylformamide (DMF) or N,N-dimethylacetamide (DMAc), or acetonitrile.

The base may, for example, be an alkali metal such as metal sodium or metal potassium, an alkali metal hydride or alkaline earth metal hydride such as sodium hydride, potassium hydride or calcium hydride, a carbonate such as sodium carbonate, potassium carbonate or calcium carbonate, or a metal hydroxide such as sodium hydroxide, potassium hydroxide or calcium hydroxide. Such a base can be used in a suitable combination with the solvent.

### Process B

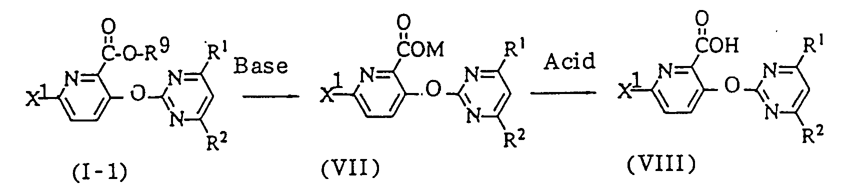

In the above formulas, R¹, R², R⁹ and X¹ are as defined above, and M is an alkali metal, an alkaline earth metal or an organic amine cation.

Namely, among compounds of the present invention, the compound of the formula (VII) can be obtained by reacting the formula (I-1) in the presence of a base in a polar solvent or water or in a solvent mixture of a polar solvent and water within a temperature range of from room temperature to the boiling point of the solvent for from a few hours to dozens of hours. The product is then precipitated with an acid to obtain a compound of the formula (VIII).

The solvent may, for example, be an alcohol solvent such as methanol or ethanol, an ether polar solvent such as 1,4-dioxane or tetrahydrofuran (THF), an aprotic polar solvent such as DMF, DMAc or dimethylsulfoxide, or acetonitrile. The base may, for example, be a carbonate such as sodium carbonate, potassium carbonate or calcium carbonate, or a metal hydroxide such as sodium hydroxide, potassium hydroxide or calcium hydroxide.

### Process C

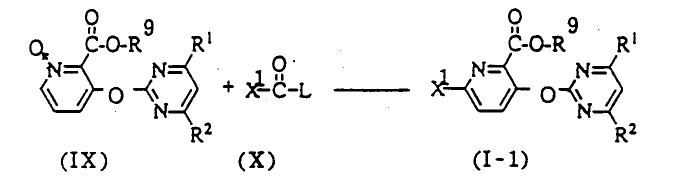

In the above formulas, R¹, R², R⁹ and X¹ are as defined above, and L is a leaving group.

Namely, the compound of the formula (IX) and the compound of the formula (X) are reacted in an inert solvent within a temperature range of from 0°C to the boiling point of the solvent for from a few minutes to dozens of hours to obtain the compound of the formula (I-1). Here, the solvent may, for example, be a hydrocarbon solvent such as toluene, benzene or xylene, a halogenated hydrocarbon solvent such as dichloromethane or chloroform, an ether solvent such as ethyl ether, isopropyl ether, tetrahydrofuran or 1,4-dioxane, an aprotic polar solvent such as DMF, DMAc or dimethylsulfoxide, an ester such as ethyl acetate, acetonitrile or chloroform. Preferred is acetonitrile or tetrahydrofuran. The solvent is used in an amount of from 0.1 to 10 ℓ, preferably from 1.0 to 5.0 ℓ, per mol of the compound of the formula (IX). As a preferred condition, refluxing in acetonitrile may be mentioned. The compound of the formula (X) may be used in an equal amount or more to the compound of the formula (IX).

In this reaction, the reaction may proceed without addition of a base. However, it is preferred to add a base. The base may, for example, be an alkali metal or an alkaline earth metal, particularly a carbonate, a hydrogencarbonate, an acetate, an alkoxide, a hydroxide or an oxide of sodium, potassium, magnesium or calcium. Further, an organic base such as pyridine or a tertiary amine such as triethylamine or N,N-diisopropylethylamine may be used. Preferred is N,N-diisopropylethylamine. The amount of the base is preferably at least an equimolar amount to the compound of the formula (X). The leaving group may, for example, be a halogen atom such as a chlorine atom, an alkoxy group, or 1-imidazolyl group. Preferred is a chlorine atom. When the leaving group is a chlorine atom, the reactivity of the compound of the formula (X) can be increased by adding potassium iodide or sodium iodide in an amount of from a catalytic amount to an equimolar amount to the compound of the formula (X).

### Process D

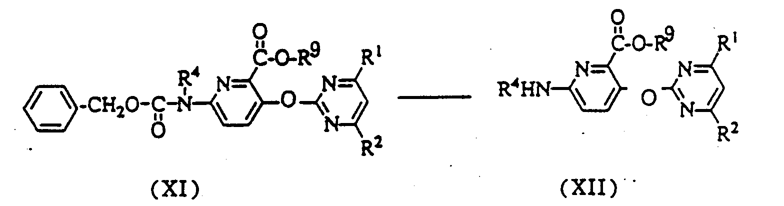

In the above formulas, R¹, R², R⁴ and R⁹ are as defined above.

Namely, the compound of the formula (XII) can be produced by catalytic hydrogenation reaction of a compound of the formula (XI) in the presence of a catalyst in an inert solvent within a temperature range of from 0°C to the boiling point of the solvent for from a few minutes to dozens of hours. The solvent may be the same as in Process A (except a halogenated hydrocarbon solvent), and the catalyst may, for example, be a reducing metal catalyst such as palladium carbon or Raney nickel. Further, depending upon the reaction, the reaction may be accelerated by an addition of a small amount of an acid such as acetic acid, sulfuric acid or a perchloric acid. As a preferred embodiment, a method may be mentioned in which the hydrogenation is conducted in a methanol solvent at room temperature in the presence of palladium carbon as a catalyst.

The compound of the formula (XI) of the present invention can be produced also by Process C as described above.

### Process E

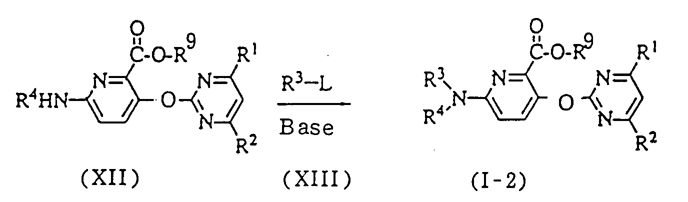

In the above formulas, R¹, R², R³, R⁴ and R⁹ are as defined above, and L is a leaving group.

Namely, the compound of the formula (I-2) of the present invention can be prepared by reacting the compound of the formula (XII) and the compound of the formula (XIII) in the presence of a base in an inert solvent within a temperature range of from 0°C to the boiling point of the solvent for from a few minutes to dozens of hours.

The solvent and the base may be the same as in Process A.

### Process F

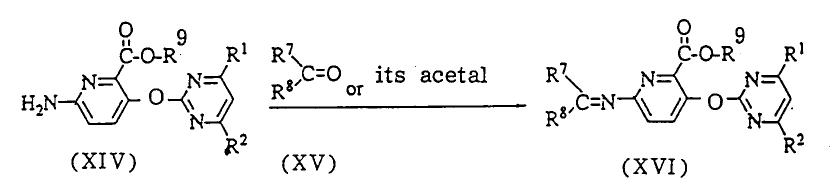

In the above formulas, R¹, R², R⁷, R⁸ and R⁹ are as defined above.

Namely, the compound of the formula (XVI) of the present invention can be produced by reacting the compound of the formula (XIV) and the compound of the formula (XV) within a temperature range of from 0°C to the boiling point of the compound of the formula (XV) for from a few minutes to dozens of hours. This reaction may be conducted by an addition of an inert solvent, and the solvent for this purpose may be the same as in Process A. Further, the reaction may be accelerated, for example, by an addition of a small amount of a Lewis acid as an acid catalyst, by dehydration operation by means of a Deanstark tube or by dehydration operation by means of a molecular sieve. However, as a preferred embodiment, a reaction under reflux in a methanol solvent may be mentioned.

### Process G

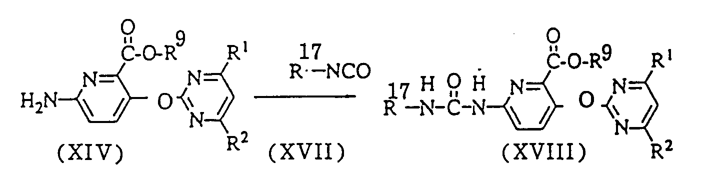

In the above formulas, R¹, R² and R⁹ are as defined above, and R¹⁷ is an alkyl group, a phenyl group or a benzyl group.

Namely, the compound of the formula (XVIII) can be produced by reacting the compound of the formula (XIV) and the compound of the formula (XVII) in an inert solvent within a temperature range of from 0°C to the boiling point of the solvent for from a few minutes to dozens of hours.

The solvent to be used here may be the same as in Process A. For this reaction, a base may be used as a catalyst. Such a base may be the same as in Process A. Further, when the compound of the formula (XVII) is liquid, the reaction can be conducted without using the inert solvent. As a preferred embodiment, a reaction may be mentioned in which an organic amine such as triethylamine is added in MEK or a halogenated hydrocarbon solvent.

### Process H

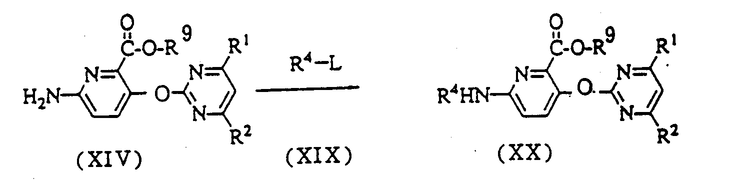

In the above formulas, R¹, R², R³, R⁹ and L are as defined above.

Namely, the compound of the formula (XX) of the present invention can be produced by reacting the compound of the formula (XIV) and the compound of the formula (XIX) in the presence of a base within a temperature range of from 0°C to the boiling point of the solvent for a few minutes to dozens of hours.

The solvent and the base to be used here may be the same as in Process A.

### Process I

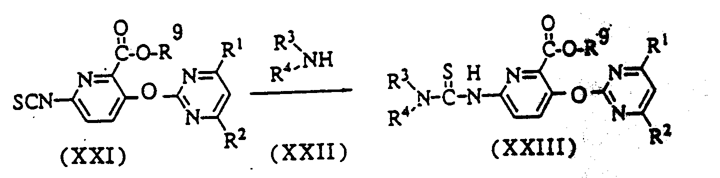

In the above formulas, R¹, R², R³, R⁴ and R⁹ are as defined above.

Namely, the compound of the formula (XXIII) of the present invention can be prepared by reacting the compound of the formula (XXI) and the compound of the formula (XXII) in an inert solvent within a temperature range of from 0°C to the boiling point of the solvent from a few minutes to dozens of hours.

The solvent to be used here, may be the same as in Process A. In this reaction, a base may be used as a catalyst, and such a base may be the same as in Process A. Further, when the compound of the formula (XXIII) is liquid, the reaction can be conducted without using the inert solvent. As a preferred embodiment, a reaction in a halogenated hydrocarbon solvent may be mentioned.

Synthesis of the compound of the formula (XXII) is described in Preparation Example 19.

### Process J

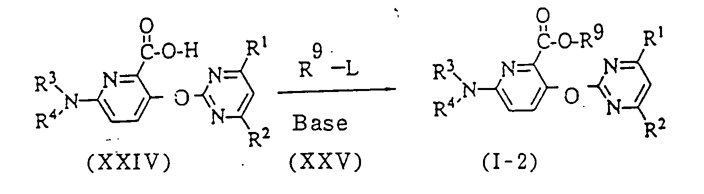

In the above formulas, R¹, R², R³, R⁴, R⁹ and L are as defined above.

Namely, the compound of the formula (I-2) of the present invention can be produced by reacting the compound of the formula (XXIV) and the compound of the formula (XXV) in the presence of a base in an inert solvent within a temperature range of from 0°C to the boiling point of the solvent for from a few minutes to dozens of hours.

The solvent and the base to be used here may be the same as in Process A.

### Process K

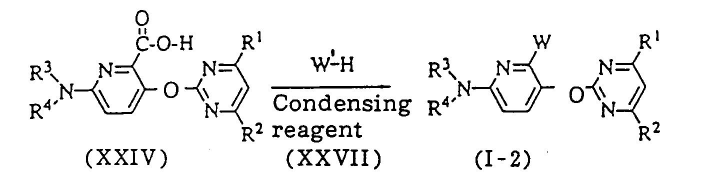

In the above formulas, W¹ is R¹⁰O, R¹¹S or R¹²R¹³N, and R¹, R², R³, R⁴, R¹⁰, R¹¹, R¹², R¹³ and W are as defined above.

Namely, the compound of the formula (I-2) of the present invention can be produced by reacting the compound of the formula (XXVI) and the compound of the formula (XXVII) in the presence of a condensing reagent in a inert solvent within a temperature range of from 0°C to the boiling point of the solvent for from a few minutes to dozens of hours.

The solvent to be used here may be the same as in Process A. The condensing reagent may be a carbonyldiimidazole or a combination thereof with a base, a combination of triphenylphosphine and diethylazodicarboxylate, diethylcyanophosphate, or an organic base. For the above two cases, the solvent is preferably THF or DMF.

Now, the processes as well as the formulations and use of the present invention will be described in more detail with Preparation Examples.

### EXAMPLE 1

### Preparation of methyl 3-[(4,6-dimethoxypyrimidin-2-yl)oxy]-6-(1-pyrrolidinyl)picolinate (Compound No. 317)

1.55 g (7 mmol) of methyl 3-hydroxy-6-(1-pyrrolidinyl)picolinate, 1.83 g (8.4 mmol) of 4,6-dimethoxy-2-methylsulfonylpyrimidine and 0.96 g (7 mmol) of potassium carbonate were added to 50 ml of DMF and reacted at 100°C for 3 hours. After completion of the reaction, the reaction product was poured into ice water, extracted with ethyl acetate, washed with a saturated sodium chloride aqueous solution and dried over magnesium sulfate. The solvent was distilled off, and the residue was crystallized from diisopropyl ether to obtain the desired product.

Amount: 2.11 g (yield: 84%), melting point: 151-153°C

### EXAMPLE 2

### Preparation of 3-[(4,6-dimethoxypyrimidin-2-yl)oxy]-6-(N-methyl-N-cyclohexylamino)picolinic acid (Compound No. 21)

6.2 g (15 mmol) of methyl 3-[(4,6-dimethoxypyrimidin-2-yl)oxy]-6-(N-methyl-N-cyclohexylamino)picolinate was dissolved in 60 ml of methanol. To this solution, 1.1 g (20 mmol) of potassium hydroxide dissolved in 30 ml of water, was added, and the mixture was reacted at 50°C for one hour. The reaction solution was concentrated and then adjusted to pH7 by an addition of a saturated citric acid aqueous solution. Then, it was extracted with chloroform and then dried and concentrated to obtain crude crystals, which were recrystallized from ethanol to obtain the desired product.

Amount: 3.6 g (yield: 60.0%), melting point: 144-147°C

### EXAMPLE 3

### Preparation of 3-[(4,6-dimethoxypyrimidin-2-yl)oxy]-6-(1-pyrrolidinyl)picolinic acid (Compound No. 318)

1.44 g (4 mmol) of methyl 3-[(4,6-dimethoxypyrimidin-2-yl)oxy]-6-(1-pyrrolidinyl)picolinate and 0.67 g (12 mmol) of potassium hydroxide were added to 30 ml of methanol, 30 ml of 1,2-dimethoxyethane and 10 ml of water, and the mixture was reacted at 40°C for 4 hours. The solvent was distilled off, and water was added to the residue. The mixture was acidified (pH=4) by 10% hydrochloric acid, then extracted with chloroform and ethyl acetate, dried and concentrated, and then crystallized from diisopropyl ether to obtain the desired product.

Amount: 1.29 g (yield: 94%), melting point: 179-183°C

### EXAMPLE 4

### Preparation of methyl 3-[(4,6-dimethoxypyrimidin-2-yl)oxy]-6-(N-methyl-N-cyclohexylamino)picolinate (Compound No. 20)

11.0 g (36 mmol) of methyl 3-[(4,6-dimethoxypyrimidin-2-yl)oxy]picolinate N-oxide, 9.5 g (54 mmol) of N-methyl-N-cyclohexylcarbamoyl chloride, 10.5 g (70 mmol) of sodium iodide and 9.0 g (70 mmol) of diisopropylethylamine were added to 150 ml of acetonitrile and reacted at a temperature of from 60 to 70°C for one hour. Water was added to the reaction solution, and the organic layer was extracted with ethyl acetate, dried and concentrated. The obtained oily product was purified by silica gel column chromatography (ethyl acetate-hexane) to obtain the desired product. Amount: 7.2 g (yield: 50%), melting point: 116-117.5°C

### EXAMPLE 5

### Preparation of methyl 3-[(4,6-dimethoxypyrimidin-2-yl)oxy]-6-(N-methyl-N-benzylamino)picolinate (Compound No. 26)

20 g (77.1 mmol) of methyl 3-[(4,6-dimethoxypyrimidin-2-yl)oxy]picolinate N-oxide, 15 g (116 mmol) of diisopropylethylamine and 46.2 g (308.2 mmol) of sodium iodide were suspended in 200 ml of acetonitrile and stirred at room temperature for 15 minutes. To this mixture, 21.2 g (115.4 mmol) of N-methyl-N-benzylcarbamoyl chloride dissolved in 20 ml of acetonitrile was added at room temperature. The mixture was refluxed. Foaming started at a temperature slightly lower than the boiling point, and such foaming terminated in about 30 minutes. The reaction was continued for further 10 minutes. The reaction solution was poured into water and extracted with ethyl acetate. The extract was washed with water, dried, concentrated and then purified by silica gel column chromatography to obtain the desired product.

Amount: 9.0 g (yield: 32%), melting point: 73-75°C

### EXAMPLE 6

### Preparation of methyl 3-[(4,6-dimethoxypyrimidin-2-yl)oxy]-6-[2-(phenoxy)ethylamino]picolinate (Compound No. 64)

1.12 g (2 mmol) of methyl 3-[(4,6-dimethoxypyrimidin-2-yl)oxy]-6-[N-(2-phenoxy)ethyl-N-benzyloxycarbonylamino]picolinate was catalytically reduced in 30 ml of methanol in the presence of 1 g of palladium carbon (10%). The catalyst was filtered off, and the filtrate was distilled under reduced pressure at a low temperature of not higher than 40°C, and the residual oily product was purified by column chromatography to obtain the desired product as colorless transparent oily product.

Amount: 0.6 g (yield: 70.4%), refractive index: 1.5682

### EXAMPLE 7

### Preparation of methyl 3-[(4,6-dimethoxypyrimidin-2-yl)oxy]-6-(N-methoxymethyl-N-methyl)aminopicolinate (Compound No. 77)

2.1 g (6.6 mmol) of methyl 3-[(4,6-dimethoxypyrimidin-2-yl)oxy]-6-methylaminopicolinate and 1.30 g (10.0 mmol) of N,N-diisopropylethylamine were added to 20 ml of dichloromethane, and 0.80 g (10.0 mmol) of methoxymethyl chloride was added thereto at room temperature. The mixture was left to stand overnight, and then dichloromethane was distilled off under reduced pressure. The residue was purified by silica gel column chromatography to obtain the desired product.

Amount: 0.30 g (yield: 12.5%)

### EXAMPLE 8

### Preparation of methyl 3-[(4,5-dimethoxypyrimidin-2-yl)oxy]-6-(N,N-dimethylaminomethyleneamino)picolinate (Compound No. 321)

2.0 g (6.5 mmol) of methyl 6-amino-3-[(4,6-dimethoxypyrimidin-2-yl)oxy]picolinate and 1.5 g (10 mmol) of N,N-dimethylformamide diethylacetal were added to 20 ml of ethanol, and the mixture was stirred and refluxed for two hours. After completion of the reaction, the solvent was distilled off under reduced pressure. The residue was purified by column chromatography to obtain the desired product as colorless transparent prism crystals.

Amount: 1.5 g (yield: 65.2%), melting point: 133-137°C

### EXAMPLE 9

### Preparation of methyl 3-[(4,6-dimethoxypyrimidin-2-yl)oxy]-6-(N-phenylcarbamoylamino)picolinate (Compound No. 181)

2 g (6.5 mmol) of methyl 6-amino-3-[(4,6-dimethoxypyrimidin-2-yl)oxy]picolinate, 0.77 g (7.15 mmol) of phenyl isocyanate and a catalytic amount of crystals of 1,4-diazabicyclo-[2.2.2]-octane were added to 50 ml of MEK, and the mixture was stirred and refluxed for 4 hours. After completion of the reaction, the product was filtered and washed with MEK to obtain the desired product as colorless transparent crystals.

Amount: 1.97 g (yield: 71.1%), melting point: 236-237°C

### EXAMPLE 10

### Preparation of methyl 3-[(4,6-dimethoxypyrimidin-2-yl)oxy]-6-[N-(1,1,1-trifluoroacetyl)amino]picolinate (Compound No. 146)

5.8 g (27.4 mmol) of methyl 6-amino-3-[(4,6-dimethoxypyrimidin-2-yl)oxy]picolinate, 7.6 g (24.9 mmol) of trifluoroacetic anhydride and 2.6 g (32.4 mmol) of pyridine were added to 60 ml of MEK, and the mixture was stirred at room temperature for one hour. After completion of the reaction, the reaction mixture was poured into a dilute alkaline aqueous solution, then extracted with ethyl acetate. The extract was washed with water, dried and concentrated. Then, obtained crude crystals were recrystallized from ethanol to obtain the desired product as colorless transparent crystals.

Amount: 7.7 g (yield: 77%), melting point: 138-140°C

### EXAMPLE 11

### Preparation of methyl 3-[(4,6-dimethoxypyrimidin-2-yl)oxy]-6-(methylsulfonylamino)picolinate (Compound No. 300)

3.1 g (10 mmol) of methyl 3-[(4,6-dimethoxypyrimidin-2-yl)oxy]-6-aminopicolinate, 2.4 g (21 mmol) of methanesulfonyl chloride and 1.5 g (11 mmol) of potassium carbonate were added to 10 ml of MEK, and the mixture was stirred and refluxed for 12 hours. After completion of the reaction, the solvent was distilled off under reduced pressure. The residual oily product was purified by column chromatography and then crystallized from diisopropyl ether to obtain the desired product as a slightly yellow powder.

Amount: 0.75 g (yield: 19.7%), melting point: 144-146°C

### EXAMPLE 12

### Preparation of methyl 6-(2-butenylamino)-3-[(4,6-dimethoxypyrimidin-2-yl)oxy]picolinate (Compound No. 56)

3.0 g (9.8 mmol) of methyl 6-amino-3-[(4,6-dimethoxypyrimidin-2-yl)oxy]picolinate, 1.5 g (10.8 mmol) of 2-butenyl bromide and 1.6 g (11.8 mmol) of potassium carbonate were added to 10 ml of DMF, and the mixture was stirred at 100°C for one hour. After completion of the reaction, the reaction mixture was poured into water, then extracted with ethyl acetate, dried and concentrated. Then, the obtained residue was purified by column chromatography and crystallized to obtain a solid, which was washed with diisopropyl ether to obtain the desired product as colorless prism crystals.

Amount: 1.28 g (yield: 36.6%), melting point: 82-85°C

### EXAMPLE 13

### Preparation of methyl 3-[(4,6-dimethoxypyrimidin-2-yl)oxy]-6-(N-n-propylthiocarbamoylamino)picolinate (Compound No. 265)

2 g (5.7 mmol) of methyl 3-[(4,6-dimethoxypyrimidin-2-yl)oxy]-6-(isothiocyanate)picolinate and 0.4 g (6.8 mmol) of n-propylamine were added to 50 ml of dichloromethane, and the mixture was stirred at room temperature for 12 hours. After completion of the reaction, the product was filtered and washed with dichloromethane to obtain the desired product as colorless transparent crystals.

Amount: 2.2 g (yield: 94%), melting point: 218-220°C

### EXAMPLE 14

### Preparation of isopropylideneamino 6-amino-3-[(4,6-dimethoxypyrimidin-2-yl)oxy]picolinate (Compound No. 337)

1.5 g (5 mmol) of 6-amino-3-[(4,6-dimethoxypyrimidin-2-yl)oxy]picolinic acid, 0.8 g (5 mmol) of carbonyldiimidazole and 0.25 g (3 mmol) of N-isopropylidenehydroxylamine were stirred and refluxed in dichloromethane for 4 hours. After completion of the reaction, the reaction mixture was poured into water, and the dichloromethane layer was separated, washed with water and dried. Then, the solvent was distilled off under reduced pressure. The residual oily product was purified by column chromatography to obtain the desired product as colorless transparent crystals.

Amount: 0.7 g (yield: 58.3%), melting point: 210-214°C

### EXAMPLE 15

### Preparation of S-ethyl 6-amino-3-[(4,6-dimethoxypyrimidin-2-yl)oxy]picolinthioate (Compound No. 344)

2.5 g (8.5 mmol) of 6-amino-3-[(4,6-dimethoxypyrimidin-2-yl)oxy]picolinic acid, 0.64 g (10.3 mmol) of ethylmercaptan, 2.8 g (17 mmol) of diethyl cyano phosphate and 1.73 g (17.1 mmol) of triethylamine were added to a THF solution, and the mixture was stirred at room temperature for 4 hours. After completion of the reaction, the reaction mixture was poured into water and extracted with ethyl acetate, followed by separation, washed with water and dried. Then, the solvent was distilled off, and the residual oily product was purified by column chromatography to obtain the desired product as colorless transparent crystals.

Amount: 1.0 g (yield: 34.7%), melting point: 115-118°C

### EXAMPLE 16

### Preparation of N'-methyl-N'-methoxy-3-[(4,6-dimethoxypyrimidin-2-yl)oxy]-6-(N,N-dimethylamino)picolinamide (Compound No. 349)

4.0 g (12 mmol) of 6-(N,N-dimethylamino)-3-[(4,6-dimethoxypyrimidin-2-yl)oxy]picolinic acid and 3.0 g (19 mmol) of carbonyl diimidazole were added to 30 ml of THF, and the mixture was stirred at room temperature for 4 hours. Then, the reaction mixture was poured into water and extracted with ethyl acetate. The extract was washed with water and dried. The solvent was distilled off, and the residual oily product was crystallized from diisopropyl ether to obtain 4.0 g (yield: 87%) of 6-(N,N-dimethylamino)-3-[(4,6-dimethoxypyrimidin-2-yl)oxy]picolinoylimidazole as a reaction intermediate. 1 g (2.7 mmol) of this intermediate, 0.3 g (3.7 mmol) of N-methoxy-N-methylamine hydrochloride and 0.8 g (7.5 mmol) of sodium carbonate were added to 50 ml of acetone, and the mixture was stirred at room temperature for 30 minutes. After completion of the reaction, the reaction mixture was poured into water and extracted with ethyl acetate. The extract was washed with water, dried and concentrated. Then, the residual oily product was crystallized from diisopropyl ether to obtain the desired product as colorless prism crystals.

Amount: 0.9 g (yield: 90%), melting point: 157-161°C

### EXAMPLE 17

### Preparation of methyl 6-(N-picolinoylamino)-3-[(4,6-dimethoxypyrimidin-2-yl)oxy]picolinate (Compound No. 277)

2.0 g (6.5 mmol) of methyl 6-amino-3-[(4,6-dimethoxypyrimidin-2-yl)oxy]picolinate, 1.2 g (6.5 mmol) of picolinoyl chloride hydrochloride and 2.0 g (14.8 mmol) of potassium carbonate were added to 30 ml of MEK, and the mixture was refluxed for 6 hours under stirring. After completion of the reaction, the reaction mixture was poured into water and extracted with ethyl acetate. The extract was washed with water and dried. The solvent was distilled off to obtain crude crystals. The crude crystals were washed with diisopropyl ether-ethyl acetate to obtain the desired product as colorless needle-like crystals.

Amount: 1.1 g (yield: 40.7%), melting point: 146-150°C

### EXAMPLE 18

### Preparation of methyl 3-[(4,6-dimethoxypyrimidin-2-yl)oxy]-6-(isothiocyanato)picolinate (Compound No. 319

0.82 g (7.15 mmol) of thiophosgene was dropwise added at room temperature to a solution of 2 g (6.5 mmol) of methyl 6-amino-3-[(4,6-dimethoxypyrimidin-2-yl)oxy]picolinate in 70 ml of dichloromethane and 50 ml of water. The mixture was stirred at room temperature for two hours. After completion of the reaction, the dichloromethane layer was separated and washed with water. Then, the organic layer was dried and concentrated. The obtained residue was recrystallized from a mixture of dichloromethane and diisopropyl ether to obtain the desired product as slightly yellow transparent crystals.

Amount: 1.7 g (yield: 76.6%), melting point: 116-117°C

The starting material compounds for the compound of the present invention can be prepared in accordance with the following process P.

### Process P

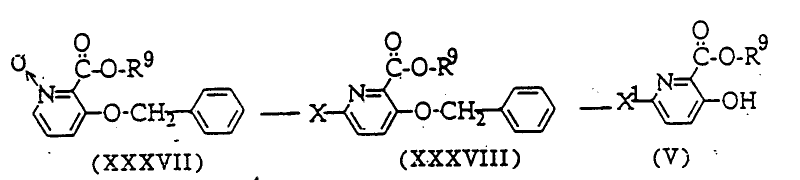

In the above formulas, R⁹ and X¹ are as defined above.

Namely, the compound of the formula (XXXVII) is converted to the compound of the formula (XXXVIII) by an N,N-dialkylcarbamoyl chloride and a base, followed by catalytic hydrogenation with hydrogen to obtain the compound of the formula (V). The production of the compound of the formula (V) from the compound of the formula (XXXVII) can be conducted also by a method disclosed in Japanese Patent Application No. 302644/1991 by the present inventors. However, such a method as disclosed in this Japanese application is not necessarily a good method, since in addition to the material necessary for the present invention, a 6-cyano compound is produced in a large amount as a by-product by the use of trimethylsilylnitrile, and its separation and purification are cumbersome.

Now, the processes for producing the starting compounds and their intermediates will be described with reference to Reference Examples.

### REFERENCE EXAMPLE 1

### Preparation of methyl 3-benzyloxy-6-(N,N-dimethylamino)picolinate (Intermediate No. 1)

1.3 g (5 mmol) of methyl 3-benzyloxypicolinate N-oxide, 3.0 g (20 mmol) of sodium iodide, 0.65 g (5 mmol) of N,N-diisopropylethylamine and 0.7 g (6.5 mmol) of N,N-dimethylcarbamoyl chloride were added to 12.5 ml of acetonitrile, and the mixture was refluxed under heating for 30 minutes. After cooling, the reaction mixture was poured into water and extracted with ethyl acetate. The organic layer was washed with water, dried and concentrated. The residue thereby obtained was purified by silica gel column chromatography to obtain the desired product.

Amount: 0.85 g (yield: 57%), melting point: 71.5-73°C

### REFERENCE EXAMPLE 2

### Preparation of methyl 6-(N,N-dimethylamino)-3-hydroxypicolinate (Intermediate No. 2)

2.3 g (8 mmol) of methyl 3-benzyloxy-6-(N,N-dimethylamino)picolinate and 0.3 g of 10% palladium carbon were added to 100 ml of ethyl acetate, and the mixture was hydrogenated under atmospheric pressure. After completion of the reaction, the product was filtered and concentrated to obtain crystals.

Amount: 1.4 g (yield: 92%), melting point: 118.5-120°C

### REFERENCE EXAMPLE 3

### Preparation of 3-benzyloxy-6-(1-pyrrolidinyl)picolinic acid

6.48 g (25 mmol) of methyl 3-benzyloxypicolinate (N-oxide), 6.68 g (50 mmol) of 1-pyrrolidinylcarbonyl chloride, 6.46 g (50 mmol) of N,N-diisopropylethylamine and 7.49 g (50 mmol) of sodium iodide were refluxed under heating for two hours in 120 ml of acetonitrile. The solvent was distilled off, and water was added to the residue. The mixture was extracted with ethyl acetate, washed with a saturated sodium chloride aqueous solution and dried over anhydrous magnesium sulfate. The solvent was distilled off, and the obtained mixture was purified by silica gel column chromatography (ethyl acetate-n-hexane) to obtain the desired product.

Amount: 6.50 g (yield: 83%), melting point: 120-121.5°C

### REFERENCE EXAMPLE 4

### Preparation of methyl 3-hydroxy-6-(1-pyrrolidinyl)picolinate (Intermediate No. 11)

6.10 g (19.5 mmol) of methyl 3-benzyloxy-6-(1-pyrrolidinyl)picolinate was catalytically reduced in 50 ml of methanol and 100 ml of 1,2-dimethoxyethane in the presence of 1.5 g of 10% palladium carbon. The catalyst was filtered off, and the solvent was distilled off, and the residue was crystallized from diisopropyl ether-n-hexane to obtain the desired product.

Amount: 4.33 g (yield: 100%), melting point: 82.5-84°C

### REFERENCE EXAMPLE 5

### Preparation of methyl 6-azido-3-benzyloxypicolinate

To an acetonitrile (70 ml) solution of 10 g (38.57 mmol) of methyl 3-benzyloxypicolinate N-oxide in a 200 ml round bottomed flask, 8.9 g (7.7 mmol) of trimethylsilylazide and 4.1 g (38.57 mmol) of N,N-dimethylcarbamoyl chloride were added, and the mixture was refluxed for 12 hours. Then, 4.4 g (38.5 mmol) of trimethylsilylazide and 2.1 g (19.3 mmol) of N,N-dimethylcarbamoyl chloride were further added, and the mixture was refluxed for 12 hours. After cooling, the reaction mixture was gradually added to an ice-cooled saturated sodium hydrogen carbonate aqueous solution. Formed crystals were collected by filtration, washed with water, then dissolved in dichloromethane, dried and concentrated, and then recrystallized from ethanol to obtain 7.0 g (yield: 64%) of the desired product.

Colorless needle-like crystals, melting point: 88-89°C

### REFERENCE EXAMPLE 6

### Preparation of methyl 6-amino-3-hydroxypicolinate (Intermediate No. 3)

To a methanol (80 ml) suspension of 7 g (24.6 mmol) of methyl 6-azido-3-benzyloxypicolinate in a 200 ml round bottomed flask, 1 g of palladium carbon (10%) was added under a nitrogen gas stream, and 7.8 g (123 mmol) of ammonium formate was further added thereto. The mixture was stirred at room temperature for 12 hours. The catalyst was filtered off, and the filtrate was concentrated and dissolved in chloroform. The solvent was washed with water, dried and concentrated. Crystals thereby formed were collected by filtration and washed with diisopropyl ether to obtain 3.57 g (yield: 86.3%) of methyl 6-amino-3-hydroxypicolinate.

Yellow needle-like crystals, melting point: 170-173°C

Examples of such intermediates will be given in Table 6.

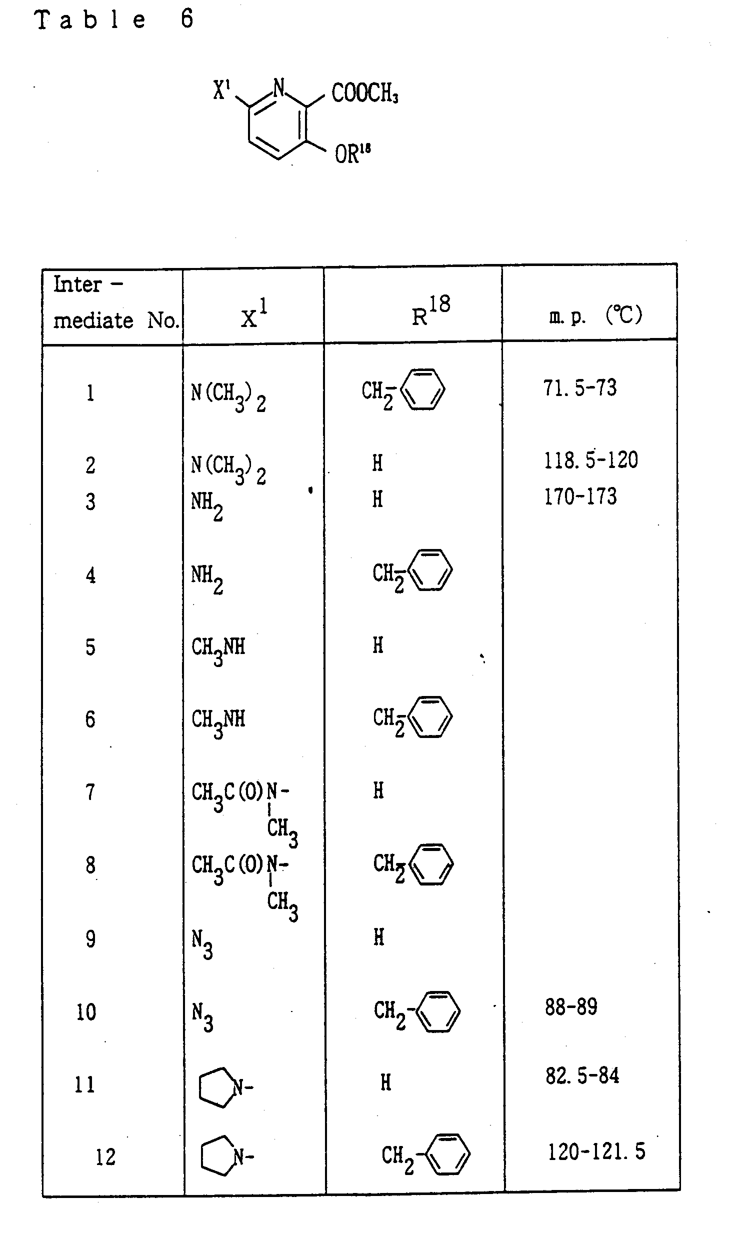

In the present invention, the starting materials of the formula (XII) and (XIV) can be prepared by the following methods.

### REFERENCE EXAMPLE 7

### Preparation of methyl 6-amino-3-[(4,6-dimethoxypyrimidin-2-yl)oxy]picolinate

4.9 g (18 mmol) of 6-acetylamino-3-benzyloxypicoline aldehyde was added to 100 ml of acetone. 400 ml of an aqueous solution of 4.3 g (27 mmol) of potassium permanganate was added thereto, and the mixture was stirred at room temperature for one hour. Precipitated manganese dioxide was filtered off and washed with 100 ml of hot water. The filtrate was extracted with ethyl acetate, and the aqueous layer was neutralized with citric acid. Precipitated crystals were extracted with chloroform, washed with water, dried and concentrated. The crystals thereby obtained were washed with diisopropyl ether to obtain 6-acetylamino-3-benzyloxy picolinic acid.

Amount: 1.5 g (yield: 29%)

1.5 g (5.2 mmol) of the obtained 6-acetylamino-3-benzyloxypicolinic acid and 1.0 g (7 mmol) of methyl iodide were dissolved in 100 ml of DMF, and 20 ml of an aqueous solution of 0.53 g (6.3 mmol) of sodium hydrogen carbonate was added thereto. The mixture was reacted at 60°C for 3 hours. The reaction solution was poured into water and extracted with ethyl acetate. The extract was washed with water, dried and concentrated. The crystals thereby obtained was washed with diisopropyl ether to obtain methyl 6-acetylamino-3-benzyloxypicolinate.

Amount: 1.57 g (yield: 90%)

1.57 g (4.7 mmol) of the obtained methyl 6-acetylamino-3-benzyloxypicolinate and 0.2 g of 10% palladium carbon were added to 100 ml of methanol, and the mixture was hydrogenated under atmospheric pressure. After completion of the reaction, the reaction mixture was filtered and concentrated to obtain crystals, which were washed with diisopropyl ether to obtain methyl 6-acetylamino-3-hydroxypicolinate.

Amount: 0.78 g (yield: 79%)

0.78 g (3.7 mmol) of the obtained methyl 6-acetylamino-3-hydroxypicolinate, 0.81 g (3.7 mmol) of 4,6-dimethoxy-2-methylsulfonylpyrimidine and 0.51 g (3.7 mmol) of potassium carbonate were added to 50 ml of DMF, and the mixture was reacted at 80°C for two hours. After completion of the reaction, the reaction mixture was poured into water and extracted with diethyl ether. The extract was washed with water, dried and concentrated. The oily product thereby obtained was purified by column chromatography to obtain methyl 6-acetylamino-3-[(4,6-dimethoxypyrimidin-2-yl)oxy]picolinate.

Amount: 0.90 g (yield: 70%), melting point: 80-85°C

0.50 g (1.4 mmol) of the obtained methyl 6-acetylamino-3-[(4,6-dimethoxypyrimidin-2- yl)oxy]picolinate was added to 50 ml of boron trifluoride-methanol complex, and the mixture was refluxed under heating for 30 minutes. The solvent was distilled off, and the residue was poured into water, neutralized with sodium hydrogen carbonate and then extracted with chloroform. The extract was washed with water, dried and concentrated. The crystals thereby obtained, were washed with ethyl acetate:hexane = 1:1 to obtain the desired product.

Amount: 0.36 g (yield: 82%), melting point: 67-69°C

### REFERENCE EXAMPLE 8

### Preparation of methyl 3-[(4,6-dimethoxypyrimidin-2-yl)oxy]-6-methylaminopicolinate

Into a photoreaction flask having a capacity of 1 ℓ, 20.0 g (60 mmol) of methyl 3-[(4,6-dimethoxypyrimidin-2-yl)oxy]-6-(N,N-dimethylamino)picolinate and 1,300 ml of chloroform were charged and irradiated by a 400W high pressure mercury lamp for 24 hours. Chloroform was distilled off under reduced pressure, and the residue was purified by silica gel column chromatography (hexane-ethyl acetate = 6:1 to 3:7) to obtain the desired product.

Amount: 2.10 g (yield: 10.7%)

### REFERENCE EXAMPLE 9

### Process for producing 3-[(4,6-dimethoxypyrimidin-2-yl)oxy]-6-methylaminopicolinic acid (First method) Preparation of methyl 6-amino-3-[(4,6-dimethoxypyrimidin-2-yl)oxy]picolinate

A DMF suspension (50 ml) of 3.0 g (17.8 mmol) of methyl 6-amino-3-hydroxypicolinate, 3.9 g (17.8 mmol) of 2-methylsulfonyl-4,6-dimethoxypyrimidine and 1.23 g (8.9 mmol) of potassium carbonate in a 100 ml round bottomed flask, was stirred at 80°C for 4 hours. The reaction mixture was poured into ice water and extracted with dichloromethane. The organic layer was washed with water, dried, concentrated and purified by silica gel chromatography (n-hexane/AcOEt = 1/1 + 0.1 MeOH) to obtain 4.43 g (yield: 81.3%) of the desired product.

Colorless prism crystals, melting point: 74-75°C

### Preparation of methyl 6-(N-acetylamino)-3-[(4,6-dimethoxypyrimidin-2-yl)oxy]picolinate

318.9 g (1.04 mol) of methyl 6-amino-3-[(4,6-dimethoxypyrimidin-2-yl)oxy]picolinate and 157.6 g (1.14 mol) of potassium carbonate were dissolved in MEK, and 89.8 g (1.14 mol) of acetyl chloride was added thereto at room temperature. The mixture was refluxed under heating (75°C) for two hours. MEK was concentrated to a half amount and then cooled. The reaction solution was poured into ice water and crystallized. Crystals thereby obtained was collected by filtration and washed with water. The crystals were dissolved in dichloromethane and subjected to liquid separation. The dichloromethane layer was dried over anhydrous magnesium sulfate and then concentrated. A small amount of diisopropyl ether were added thereto, and the mixture was cooled for crystallization. The crystals thereby obtained were recrystallized from 100 ml of ethanol. The crystals thereby obtained were washed with diisopropyl ether and then dried at 50°C for 24 hours to obtain the desired product as a white powder.

Amount: 54.3 g, melting point: 128-130.5°C, total amount 293.3 g (yield: 81%)

### Preparation of methyl 6-(N-acetyl-N-methylamino)-3-[(4,6-dimethoxypyrimidin-2-yl)oxy]picolinate

30.4 g (0.76 mol) of sodium hydride was added to DMF, and 239 g (0.69 mol) of methyl 6-N-acetylamino-3-[(4,6-dimethoxypyrimidin-2-yl)oxy]picolinate was added thereto at a temperature of at most 10°C. The mixture was stirred at room temperature for 3 hours. After confirming completion of the generation of hydrogen, 146.9 g (1.04 mol) of methyl iodide was dropwise added thereto at a temperature of at most 10°C. The mixture was returned to room temperature and then stirred for one hour. The mixture was poured into ice water and subjected to crystallization, followed by filtration. The crystals were washed with water and dried to obtain 195.6 g (yield: 78%) of the desired product.

Colorless needle-like crystals, melting point: 119-121°C

### Preparation of 3-[(4,6-dimethoxypyrimidin-2-yl)oxy]-6-methylaminopicolinic acid

195.6 g (0.54 mol) of methyl 6-(N-acetyl-N-methylamino)-3-[(4,6-dimethoxypyrimidin-2-yl)oxy]picolinate was dissolved in 1.5 ℓ of methanol. The solution was added to 335 g of a 20% potassium hydroxide aqueous solution, and the mixture was stirred for one hour at 50°C. The reaction solution was concentrated, and 1 ℓ of ice water was added thereto. The mixture was acidified (to about pH4) with an aqueous citric acid solution and left to cool. Precipitated crystals were collected by filtration and washed with cool water and diisopropyl ether. Then, the crystals were dissolved in 1 ℓ of methanol, and 45.6 g (0.69 mol) of potassium hydroxide and 50 ml of water were added thereto. The mixture was stirred at room temperature for 28 hours. The reaction solution was concentrated, and ice water was added thereto. The mixture was acidified with an aqueous citric acid solution to obtain crude crystals. The crystals were washed with diisopropyl ether and then dried under reduced pressure at 90°C for 24 hours to obtain 112 g (yield: 68%) of the desired product as a white powder.

Melting point: 172-174°C

### Process for producing 3-[(4,6-dimethoxypyrimidin-2-yl)oxy]-6-methylaminopicolinic acid (Second method)

10.5 mg (0.0576 mmol) of methyl 6-methylamino-3-hydroxypicolinate, 11.6 mg (0.0532 mmol) of 2-methylsulfonyl-4,6-dimethoxypyrimidine, 8.8 mg (0.637 mmol) of potassium carbonate and 0.5 ml of dry dimethylsulfoxide were mixed. The mixture was stirred at room temperature for 5 hours, and then a 10% potassium hydroxide aqueous solution (corresponding to 90 mg, 0.160 mmol) was added thereto. The mixture was reacted at room temperature for one hour, and then 2.0 ml of water was added to the reaction mixture. Further, 1.0 ml of a 10% citric acid aqueous solution was added thereto, and the mixture was left to stand, whereby crystals precipitated. After being thoroughly precipitated, the crystals were filtered under suction and washed with water. The crystals were dried to obtain; 3-[(4,6-dimethoxypyrimidin-2-yl)oxy]-6-methylaminopicolinic acid.

Colorless prism crystals, 13.7 mg (yield: 84.0%)

The herbicidal composition of the present invention comprises the picolinic acid derivative of the formula (I) and (II) as an active ingredient.

The compound of the present invention may be used by itself as a herbicide. However, it may be used usually in the form of a formulation such as a dust, a wettable powder, an emulsifiable concentrate, a microgranule or a granule by blending it with a carrier which is commonly used for formulations, a surfactant, a dispersant or an adjuvant.

The carrier to be used for such formulations, may, for example, be a solid carrier such as Jeaklite®, talc, bentonite, clay, kaolin, diatomaceous earth, fine silica, vermiculite, calcium carbonate, slaked lime, silica sand, ammonium sulfate or urea, or a liquid carrier such as isopropyl alcohol, xylene, cyclohexane or methylnaphthalene.

As the surfactant and dispersant, a metal salt of alkylbenzenesulfonic acid, a metal salt of dinaphthylmethane disulfonic acid, a salt of alcohol sulfuric acid ester, a salt of alkylaryl sulfonic acid, a salt of lignin sulfonic acid, a polyoxyethylene glycol ether, a polyoxyethylene alkylaryl ether or a polyoxyethylene sorbitol monoalkylate may, for example, be mentioned. The adjuvant may, for example, be carboxymethyl cellulose, polyethylene glycol or gum arabic.

In practical use, the herbicide may be diluted to a suitable concentration before application, or may be directly applied.

The herbicide of the present invention may be used for application to foliage, soil or water surface. The blending proportion of the active ingredient is suitably selected as the case requires. However, in a case of a dust or a granule, the proportion of the active ingredient is selected suitably within a range of from 0.01 to 10% by weight, preferably from 0.05 to 5% by weight. In a case of an emulsifiable concentrate or a wettable powder, the proportion is selected suitably within a range of from 1 to 50% by weight, preferably from 5 to 20% by weight.

The dose of the herbicide of the present invention varies depending upon the type of the compound, the weeds to be controlled, the germination tendency, the environmental conditions and the type of the formulation to be used. However, in the case of a dust or a granule which is used by itself, the dose of the active ingredient is selected suitably within a range of from 0.1 g to 5 kg, preferably from 1 g to 1 kg, per 10 ares. In a case of an emulsifiable concentrate or a wettable powder which is used in a liquid state, the dose of the active ingredient is selected suitably within a range of from 1 to 50,000 ppm, preferably from 10 to 10,000 ppm.

Further, the compound of the present invention may be used in combination with an insecticide, a fungicide, other herbicide, a plant growth controlling agent, a fertilizer or the like, as the case requires.

Now, the formulation method will be described with reference to typical Formulation Examples. In the following description, "parts" means "parts by weight".

### FORMULATION EXAMPLE 1 (Wettable powder)

To 10 parts by weight of Compound No. 14, 0.5 part of polyoxyethyleneoctylphenyl ether, 0.5 part of sodium β-naphthalene sulfonate-formalin condensate, 20 parts of diatomaceous earth and 69 parts of clay were mixed and pulverized to obtain a wettable powder.

### FORMULATION EXAMPLE 2 (Wettable powder)

To 10 parts of Compound No. 83, 0.5 part of polyoxyethyleneoctylphenyl ether, 0.5 part of sodium β-naphthalene sulfonate-formalin condensate, 20 parts of diatomaceous earth, 5 parts of fine silica and 64 parts of clay were mixed and pulverized to obtain a wettable powder.

### FORMULATION EXAMPLE 3 (Wettable powder formulated with calcium carbonate)

To 10 parts of Compound No. 277, 0.5 part of polyoxyethyleneoctylphenyl ether, 0.5 part of sodium β-naphthalene sulfonate-formalin condensate, 20 parts of diatomaceous earth, 5 parts of fine silica and 64 parts of calcium carbonate, were mixed and pulverized to obtain a wettable powder.

### FORMULATION EXAMPLE 4 (Emulsifiable concentrate)

To 30 parts of Compound No. 346, 60 parts of a mixture comprising equal amounts of xylene and isophorone and 10 parts of a mixture comprising a polyoxyethylene sorbitol alkylate surfactant, a polyoxyethylenealkylaryl polymer and an alkylaryl sulfonate, were added, and the mixture was thoroughly stirred to obtain an emulsifiable concentrate.

### FORMULATION EXAMPLE 5 (Granule)

10 parts of Compound No. 337, 80 parts of a bulking agent comprising a 1:3 mixture of talc and bentonite, 5 parts of fine silica, 5 parts of a mixture comprising a polyoxyethylene sorbitol alkylate surfactant, a polyoxyethylenealkylaryl polymer and an alkylaryl sulfonate and 10 parts of water were mixed and thoroughly kneaded to obtain a paste, which was extruded from sieve apertures with a diameter of 0.7 mm. The extruded product was dried and then cut into a length of from 0.5 to 1 mm to obtain granules.

Now, the herbicidal effects of the compounds of the present invention will be described with reference to Test Examples.

### TEST EXAMPLE 1 (Test on herbicidal effects by paddy field soil treatment)

In a plastic pot (surface area: 100 cm²) filled with paddy field soil, barnyardgrass (Eo), monochoria (Mo) and bulrush (Sc) were sown after puddling and leveling, and flooded to a water depth of 3 cm. Next day, a wettable powder prepared in accordance with Formulation Example 1 was diluted with water and applied dropwise to the water surface. The dose was 100 g of the active ingredient per 10 ares. The plants were then cultured in a green house, and the evaluation of the herbicidal effects was conducted on the 21st day after the application in accordance with the standards as identified in Table 7. The results are shown in the following Table 8.

**Table 7**

| Index No. | Herbicidal effects (growth-controlling degree) or phytotoxicity |
|---|---|
| 5 | Herbicidal effect or phytotoxicity: at least 90% |
| 4 | Herbicidal effect or phytotoxicity: at least 70% and less than 90% |
| 3 | Herbicidal effect or phytotoxicity: at least 50% and less than 70% |
| 2 | Herbicidal effect or phytotoxicity: at least 30% and less than 50% |
| 1 | Herbicidal effect or phytotoxicity: at least 10 and less than 30% |
| 0 | Herbicidal effect or phytotoxicity: 0 to less than 10% |

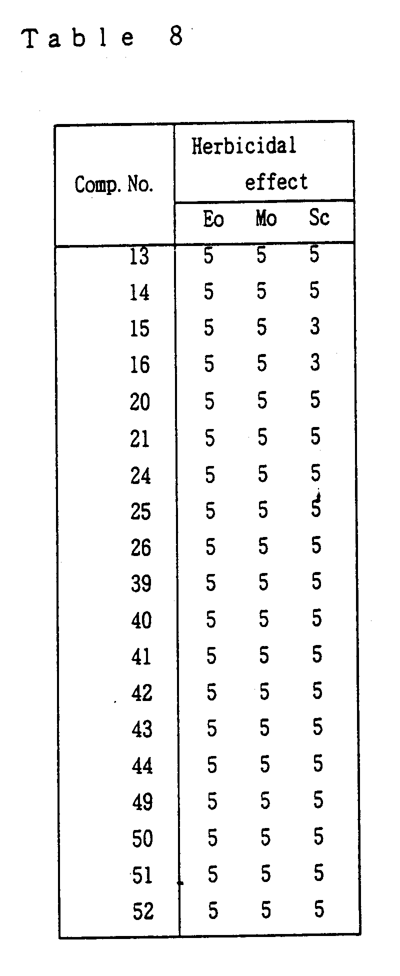

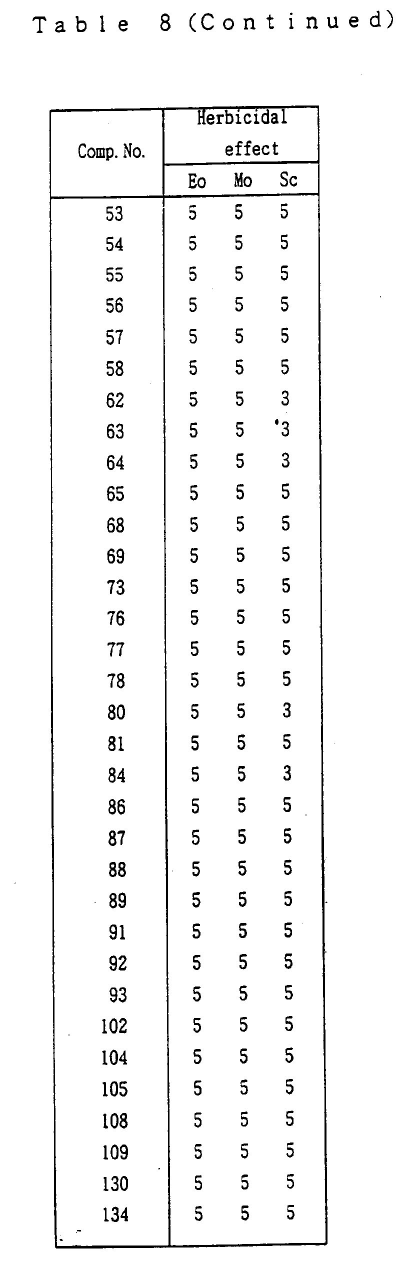

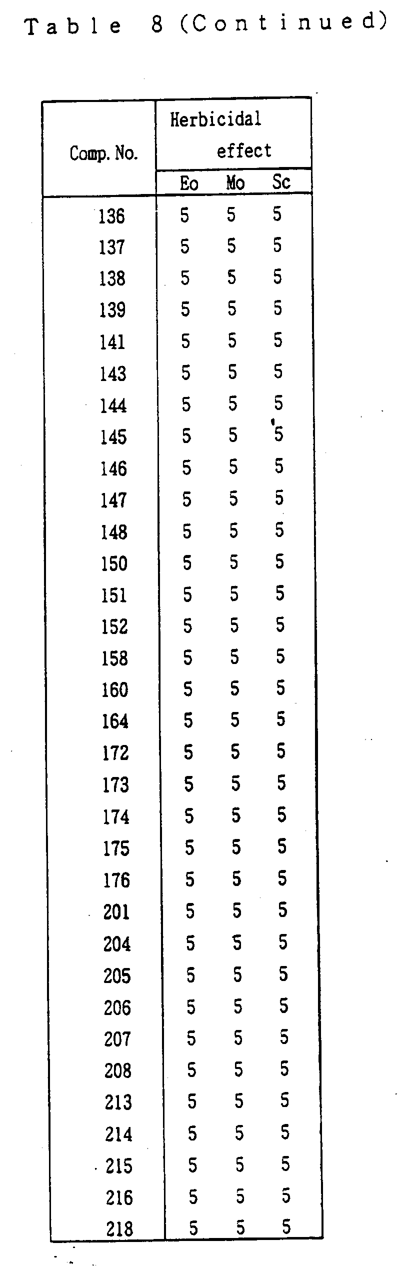

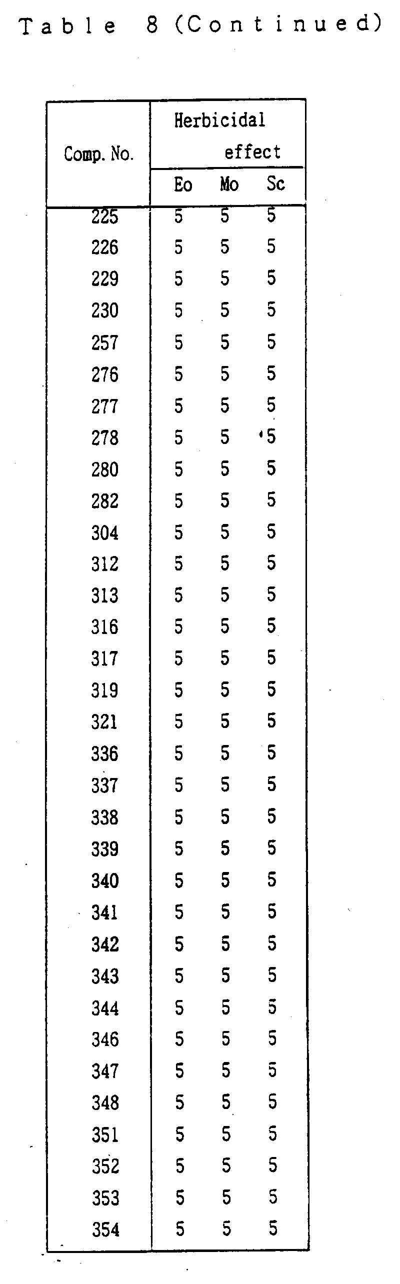

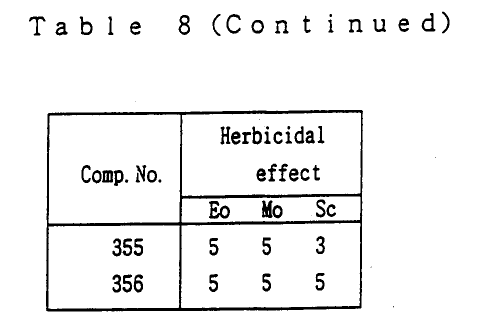

### TEST EXAMPLE 2 (Test on herbicidal effects by upland field soil treatment)

In a plastic pot (surface area: 120 cm²) filled with upland field soil, barnyardgrass (Ec), pale smartweed (Po), slender amaranth (Am), common lambsquarters (Ch) and rice flatsedge (Ci) were sown and covered with soil. A wettable powder prepared in accordance with Formulation Example 1 was diluted with water and applied uniformly to the soil surface by a small-sized sprayer in an amount of 100 ℓ/10 ares so as to apply 100 g of the active ingredient per 10 ares. The plants were then cultured in a green house, and the evaluation of the herbicidal effects was conducted on the 21st day after the application in accordance with the standards as identified in the above Table 5. The results are shown in the following Table 9.

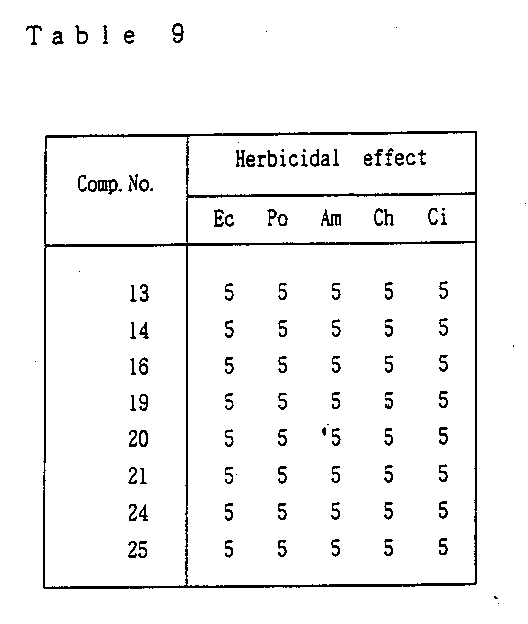

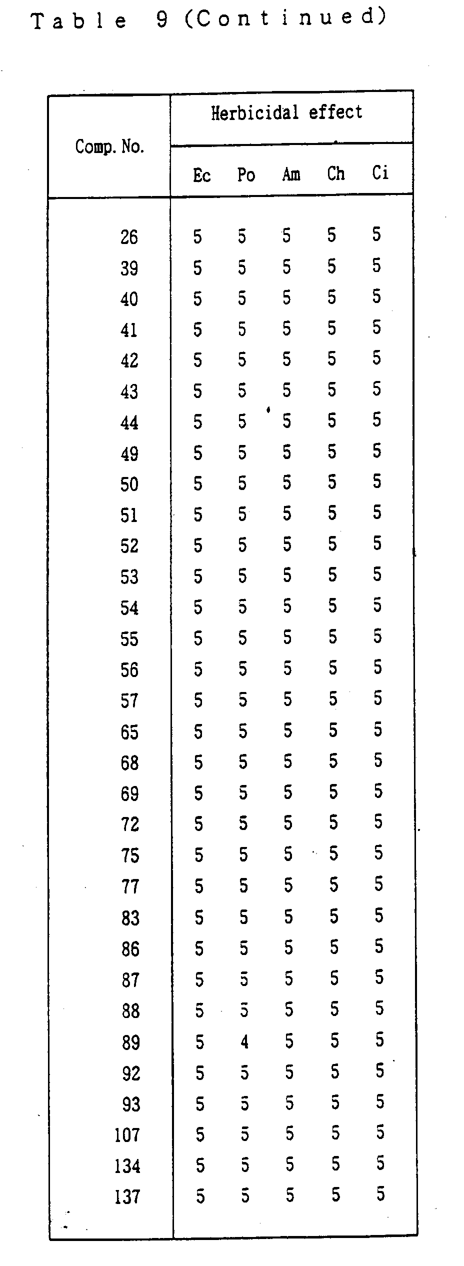

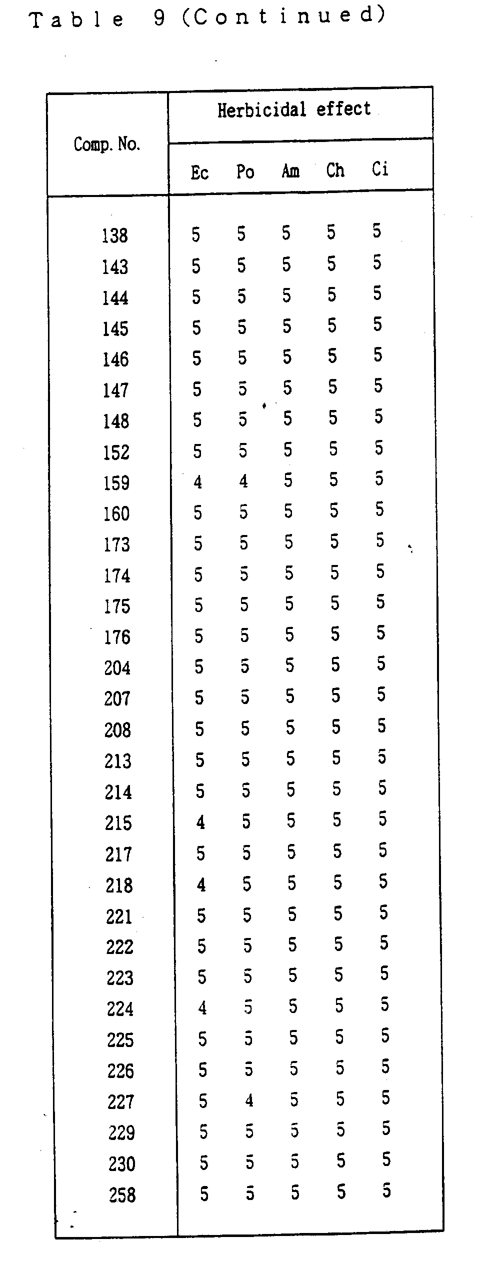

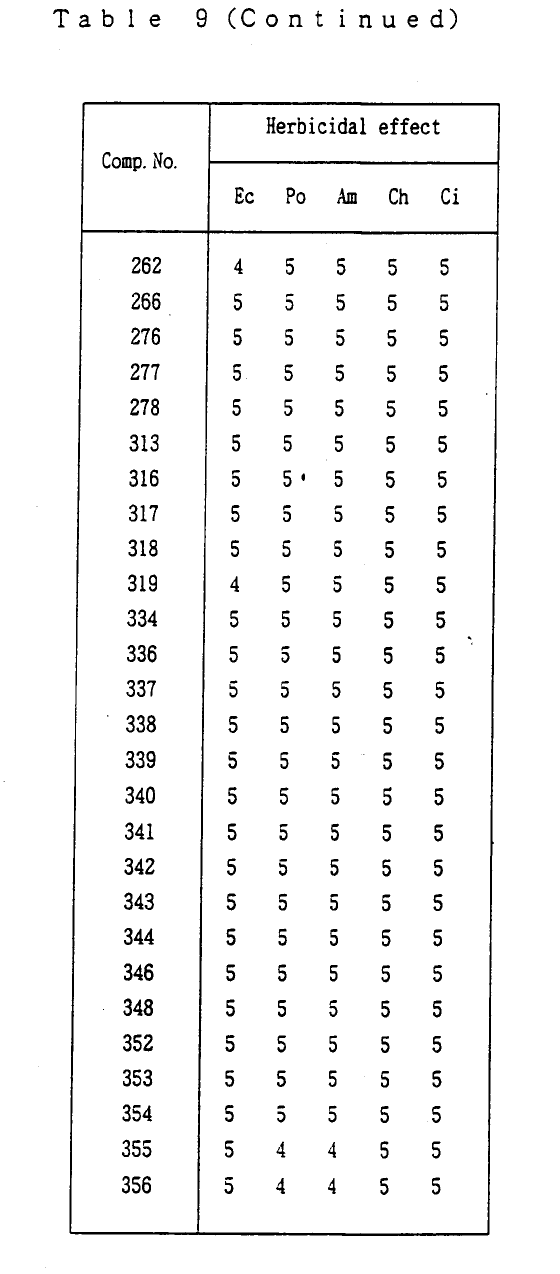

### TEST EXAMPLE 3 (Test on herbicidal effects by upland field foliage treatment)

In a plastic pot (surface area: 120 cm²) filled with upland field soil, barnyardgrass (Ec), pale smartweed (Po), slender amaranth (Am), common lambsquarters (Ch) and rice flatsedge (Ci) were sown and covered with soil and were cultured in a green house for 2 weeks. A wettable powder prepared in accordance with Formulation Example 1 was diluted with water and applied onto the entire foliages of the plants from above by a small-sized sprayer in an amount of 100 ℓ/10 ares so as to apply 100 g of the active ingredient per 10 ares. The plants were then cultured in the green house, and the evaluation of the herbicidal effects was conducted on the 14th day after the treatment in accordance with the standards as identified in the above Table 5. The results are shown in the following Table 10.

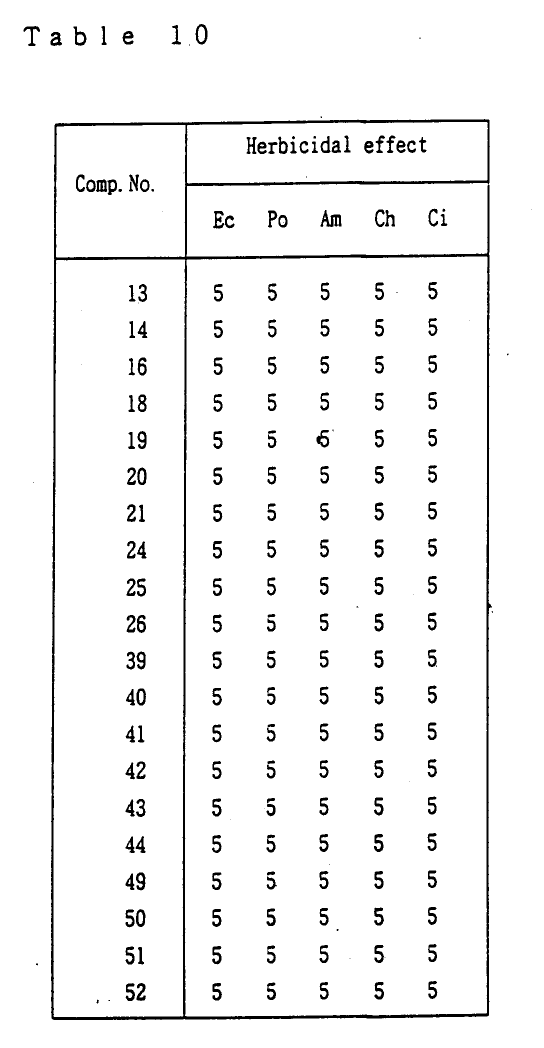

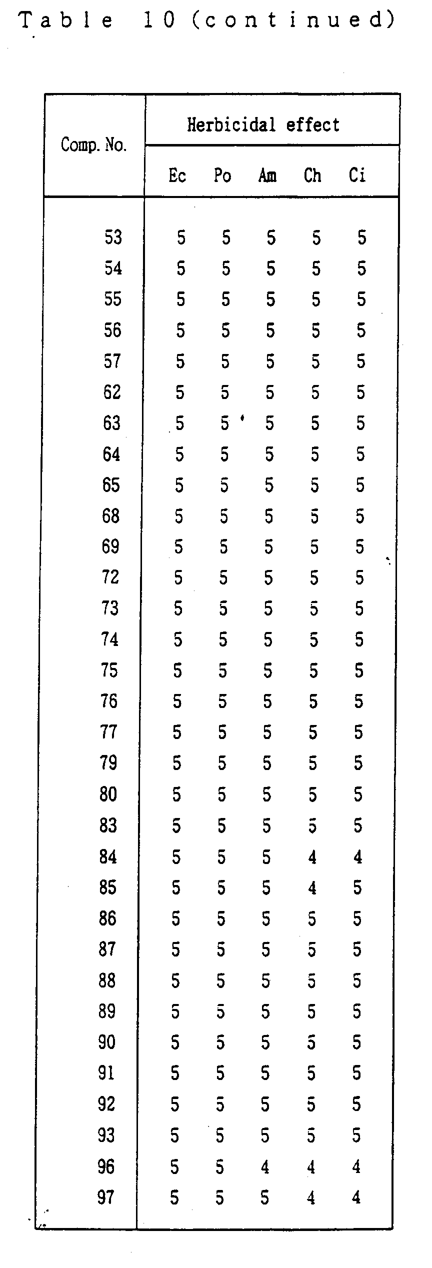

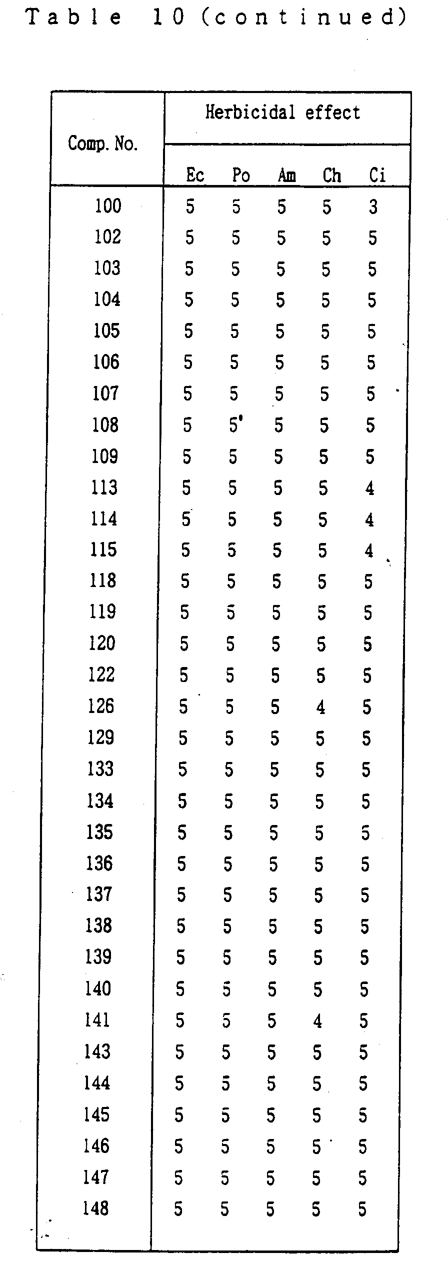

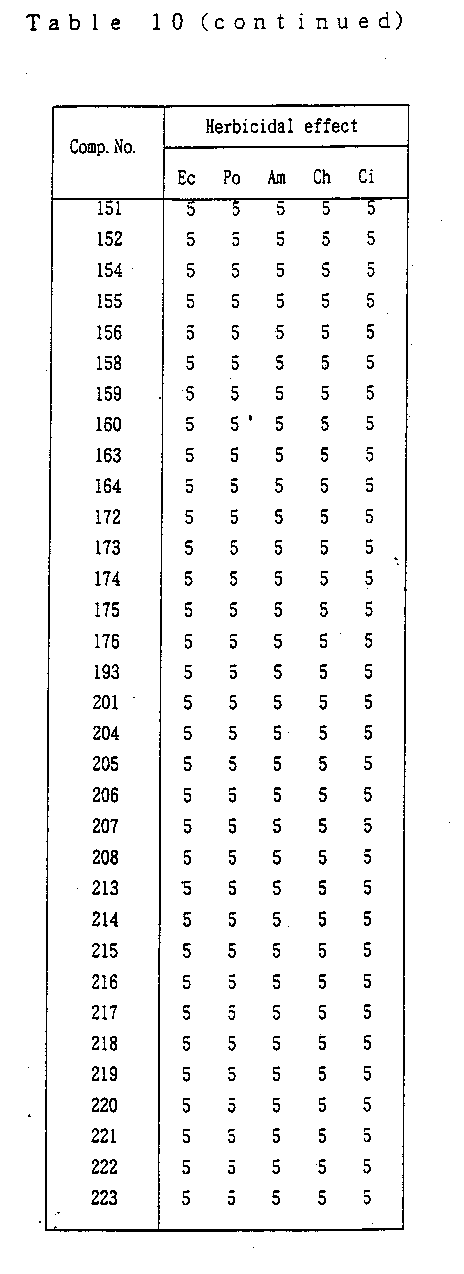

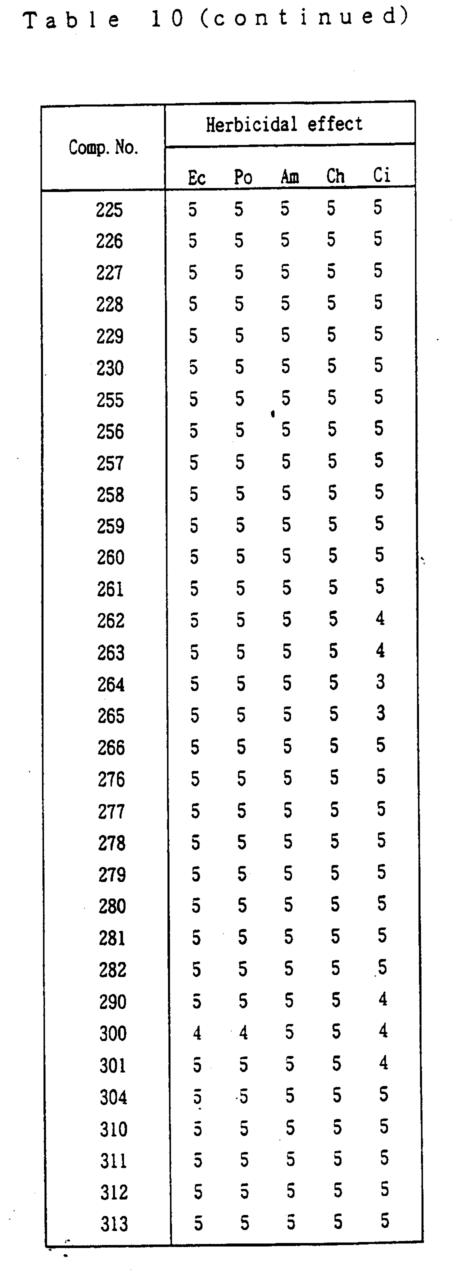

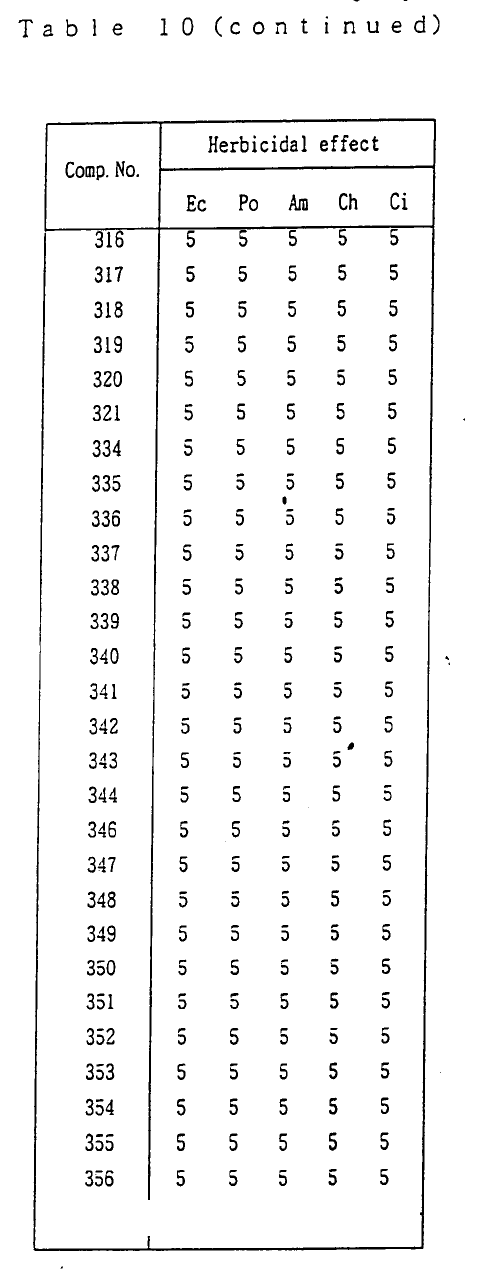

### TEST EXAMPLE 4 (Test on herbicidal effects by upland field foliage treatment at a low dose)

In a plastic pot (surface area: 600 cm²) filled with upland field soil, barnyardgrass (Ec), johnsongrass (So), pale smartweed (Po), slender amaranth (Am) and common lambsquarters (Ch) were sown and cultured in a green house for 2 weeks. A predetermined amount of a wettable powder prepared in accordance with Formulation Example 1 was diluted with water and applied onto the entire foliages of the plants from above by a small-sized sprayer in an amount of 100 ℓ/10 ares. The plants were then cultured in the green house, and the evaluation of the herbicidal effects was conducted on the 14th day after the application in accordance with the standards as identified in the above Table 5. As comparative herbicides, compounds identified below were used. The results are shown in the following Table 11.
Comparative herbicide A (Compound disclosed in PCT Patent Publication WO-9207846-A1) (Compound No. 38):
Isopropyl 3-[(4,6-dimethoxypyrimidin-2-yl)oxy]-6-(N,N-dimethylamino)picolinate
   Comparative herbicide B (Compound disclosed in PCT Patent Application WO-9207846-A1) (Compound No. 63):
Methyl 3-[(4,6-dimethoxypyrimidin-2-yl)oxy]-6-aminopicolinate

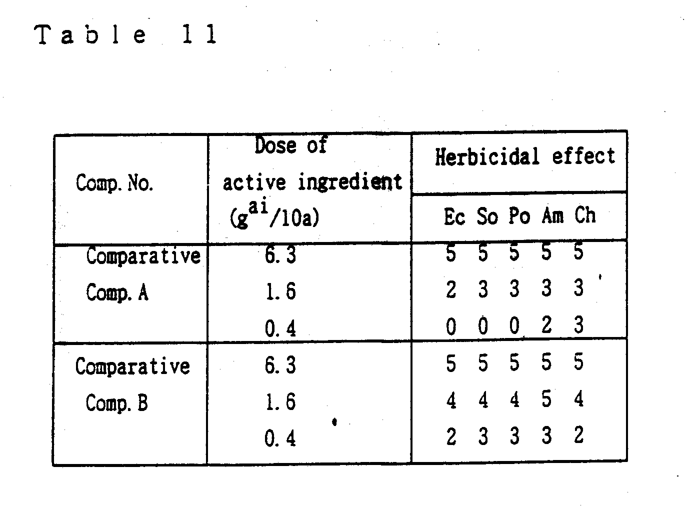

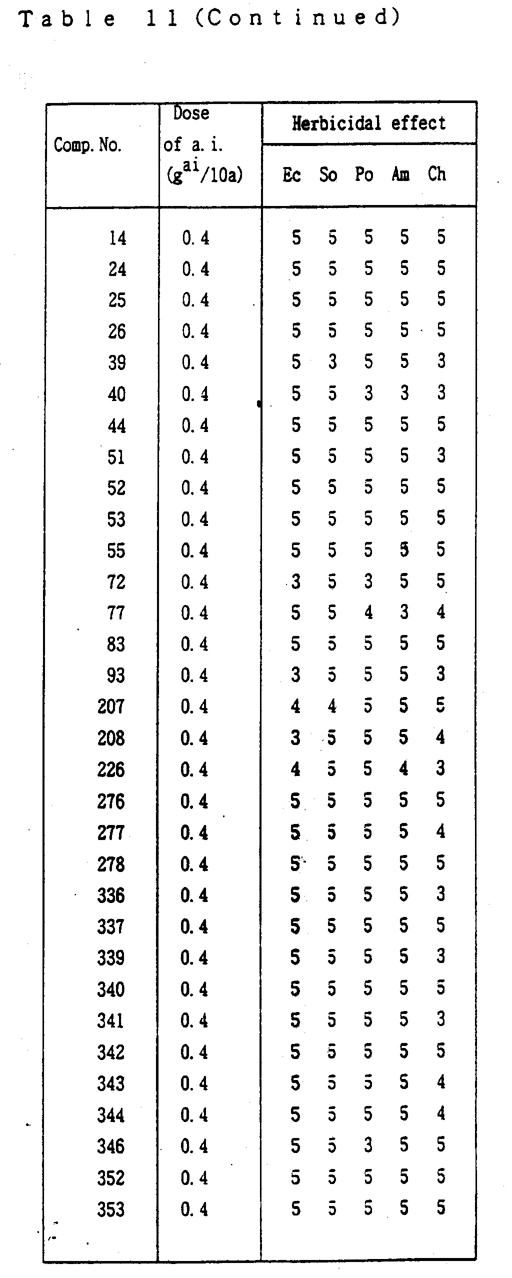

The compound of the formula (I) of the present invention has excellent herbicidal effects over a wide range of from the pre-emergence to the growing stage against various troublesome weeds grown in upland field including broadleaf weeds such as pale smartweed (Polygonumlapathifolium), slender amaranth (Amaranthus viridis), common lambsquarters (Chenopodiumalbum), chickweed (Stellaria media), velveltleaf (Abutilon theophrasti), prickly sida (Sida spinosa), America tsunokusamune (Sesbania exaltata Cory), morningglory (Pomoea sp.) and common cocklebur (Xanthumstrumarium), perennial and annual cyperaceous weeds such as purple nutsedge (Cyperus rotundus), yellow nutsedge, Kyllingabrevifolia, umbrella plant (Cyperusmicroiria) and rice flatsedge (Cyperus iria), and gramineous weeds such as barnyardgrass (Echinochloacrusgalli), crabgrass (Digitaria sp.), foxtail (Setaria sp.), green foxtail (Setariaviridis), johnsongrass (Sorghumhalepense), goosegrass (Eleusine indica) and Yaseiembaku (Avena fatua).

The compound of the present invention is also effective as a herbicide against annual weeds such as barnyardgrass (Echinochloacrusgalli), small flower flatsedge (Cyperusdifformis) and monochoria (Monochoria vaginalis), and perennial weeds such as Sagittaria pygmaea, Sagittaria trifolia, Cyperusserotinus, Eleochariskuroguwai, bulrush (Scirpushotarui) and Alismacanaliculatum, grown in paddy fields.

## Claims

1. A picolinic acid derivative of the formula: 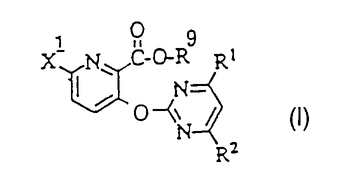 wherein each of R¹ and R² is a C₁₋₅-alkoxy group, X¹ is a group of the formula 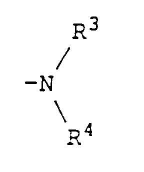 wherein R³ is a hydrogen atom, a C₁₋₅ alkyl group, a halo C₁₋₅ alkyl group, a C₁₋₅ alkoxy C₁₋₅ alkyl group, a C₂₋₆ alkenyl group, a C₂₋₆ alkynyl group, a C₁₋₅ alkylcarbonyl group, a benzyloxycarbonyl group or a phenylsulfonyl group, and R⁴ is a halo C₁₋₅ alkyl group, a C₁₋₅ alkoxy C₁₋₅ alkyl group, a C₁₋₅ alkoxy C₁₋₅ alkyloxy C₁₋₅ alkyl group, a C₁₋₅ alkoxycarbonyl C₁₋₅ alkyl group, a benzyloxy C₁₋₅ alkyl group, a C₁₋₅ alkyldioxy C₁₋₅ alkyl group, a phenoxy C₁₋₅ alkyl group, a di C₁₋₅ alkylamino C₁₋₅ alkyl group, a cyano C₁₋₅ alkyl group, a C₁₋₅ alkoxy group, a C₃₋₆ cycloalkyl group, a C₂₋₆ alkenyl group (which may be substituted at one or more positions by a halogen atom or a cyano group), a C₂₋₆ alkynyl group, a phenyl C₁₋₅ alkyl group (which may be the same or different, and substituted at one or more positions by a halogen atom, a C₁₋₅ alkyl group, a C₁₋₅ alkoxy group, a nitro group or a cyano group), a cyclo C₁₋₅ alkylcarbonyl group, a halo C₁₋₅ alkylcarbonyl group, a C₁₋₅ alkoxy C₁₋₅ alkylcarbonyl group, a cyano C₁₋₅ alkylcarbonyl group, a phenoxy C₁₋₅ alkylcarbonyl group, a phenyl C₁₋₅ alkylcarbonyl group, a halophenyl C₁₋₅ alkyl carbonyl group, a benzoyl group (which is substituted by a C₁₋₅ alkyl group, a halo C₁₋₅ alkyl group, a halogen atom, a C₁₋₅ alkoxy group, a cyano group or a nitro group), a furylcarbonyl group, a pyridylcarbonyl group, a pyrrolylcarbonyl group, a thienylcarbonyl group, a C₂₋₆ alkenylcarbonyl group, a phenyl C₂₋₆ alkenylcarbonyl group, a hydroxycarbonyl C₂₋₆ alkenylcarbonyl group, a C₁₋₅ alkoxycarbonyl C₁₋₅ alkylcarbonyl group, a C₁₋₅ alkoxy C₁₋₅ alkoxycarbonyl group, a mono C₁₋₅ alkylaminocarbonyl group, a di C₁₋₅ alkylaminocarbonyl group, a phenylaminocarbonyl group, a benzylaminocarbonyl group, a halobenzylaminocarbonyl group, a C₁₋₅ alkoxycarbonyl group, a halo C₁₋₅ alkoxycarbonyl group, a benzyloxycarbonyl group, a C₂₋₆ alkenyloxycarbonyl group, a C₂₋₆ alkynyloxycarbonyl group, a C₁₋₅ alkylsulfonyl group, a halo C₁₋₅ alkylsulfonyl group, a benzylsulfonyl group which may be substituted by a C₁₋₅ alkoxycarbonyl group, a phenylsulfonyl group which may be substituted by a C₁₋₅ alkoxycarbonyl group, a halophenylsulfonyl group, a C₁₋₅ alkylthiocarbonyl group, a halo C₁₋₅ alkylthiocarbonyl group, a benzylthiocarbonyl group, a halobenzylthiocarbonyl group, a C₂₋₆ alkenylthiocarbonyl group, a C₂₋₆ alkynylthiocarbonyl group which may be substituted by a cyano group, a C₁₋₅ alkylamino(thiocarbonyl) group, a phenylamino(thiocarbonyl) group, a di C₁₋₅ alkylamino(thiocarbonyl) group, a benzyloxy group or a group of the formula 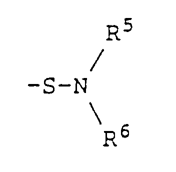 wherein each of R⁵ and R⁶ which may be the same or different, is a hydrogen atom, a C₁₋₅ alkyl group or a C₁₋₅ alkoxycarbonyl group, or R⁵ and R⁶ form together with the adjacent nitrogen atom a morpholino group, or R³ and R⁴ form together with the adjacent nitrogen atom, an azido group, an isothiocyanate group, a phthalimide group, a maleimide group, a succinimido group, a pyrrolidinyl group, a piperidino group, a pyrrolyl group, a morpholino group, a group of the formula 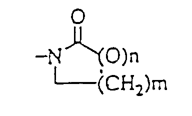 wherein n is 0 or 1, and m is 1 or 2, or a group of the formula 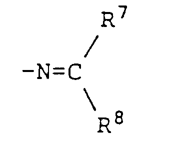 wherein each of R⁷ and R⁸ which may be the same or different, is a hydrogen atom, a C₁₋₅ alkyl group, a di C₁₋₅ alkylamino group, an amino group, a C₁₋₅ alkylthio group, a phenyl group or a benzyl group, and R⁹ is a hydrogen atom, a C₁₋₅ alkyl group, a C₂₋₆ alkenyl group, a benzyl group, an alkali metal atom, an alkaline earth metal atom or an organic amine cation; or a salt thereof.

2. A herbicidal composition comprising a herbicidally effective amount of a picolinic acid derivative of the formula (I) or a salt thereof as defined in Claim 1 and an agricultural adjuvant.

3. A picolinic acid derivative of the formula: 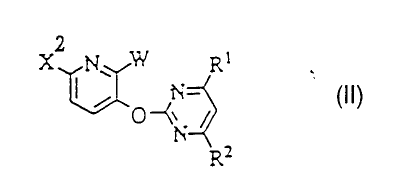 wherein each of R¹ and R² is a C₁₋₅ alkoxy group, X² is an amino group, a C₁₋₅ alkylamino group or a di C₁₋₅ alkylamino group, and W is COOR¹⁰, COSR¹¹ or 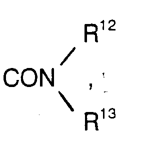 wherein R¹⁰ is a phenyl group (which may be substituted by a halogen atom or a methyl group), a benzyloxy C₁₋₅ alkyl group, a C₃₋₁₂ alkylideneamino group, a C₃₋₈ cycloalkylideneamino group or a di C₁₋₅ alkylamino group, R¹¹ is a C₁₋₅ alkyl group or a phenyl group, and each of R¹² and R¹³ which may be the same or different, is a hydrogen atom, a C₁₋₅ alkylsulfonyl group, a C₁₋₅ alkoxy C₁₋₅ alkyl group, a C₁₋₅ alkyl group, a benzyloxy group or a C₁₋₅ alkoxy group, or R¹² and R¹³ form together with the adjacent nitrogen atom, an imidazolyl group; or a salt thereof.

4. A herbicidal composition comprising a herbicidally effective amount of a picolinic acid derivative of the formula (II) or a salt thereof as defined in Claim 3 and an agricultural adjuvant.

## Patentansprüche

1. Picolinsäure-Derivat der Formel: 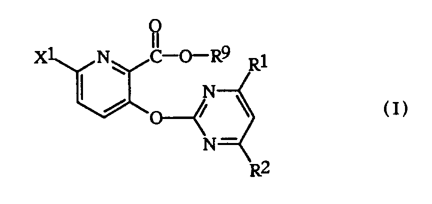 worin jedes von R¹ und R² eine C₁₋₅-Alkoxygruppe ist, X¹ eine Gruppe der Formel 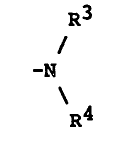 ist, worin R³ ein Wasserstoffatom, eine C₁₋₅-Alkylgruppe, eine C₁₋₅ Halogenalkylgruppe, eine C₁₋₅-Alkoxy-C₁₋₅-alkylgruppe, eine C₂₋₆-Alkenylgruppe, eine C₂₋₆-Alkinylgruppe, eine C₁₋₅-Alkylcarbonylgruppe, eine Benzyloxycarbonylgruppe oder eine Phenylsulfonylgruppe ist und R⁴ eine C₁₋₅-Halogenalkylgruppe, eine C₁₋₅-Alkoxy-C₁₋₅-alkylgruppe, eine C₁₋₅-Alkoxy-C₁₋₅-alkyloxy-C₁₋₅-alkylgruppe, eine C₁₋₅-Alkoxycarbonyl-C₁₋₅-alkylgruppe, eine Benzyloxy-C₁₋₅-alkylgruppe, eine C₁₋₅-Alkyldioxy-C₁₋₅-alkylgruppe, eine Phenoxy-C₁₋₅-alkylgruppe, eine Di-C₁₋₅-alkylamino-C₁₋₅-alkylgruppe, eine C₁₋₅-Cyanalkylgruppe, eine C₁₋₅-Alkoxygruppe, eine C₃₋₆-Cycloalkylgruppe, eine C₂₋₆-Alkenylgruppe (die an ein oder mehreren Stellen durch ein Halogenatom oder eine Cyangruppe substituiert sein kann), eine C₂₋₆-Alkinylgruppe, eine Phenyl-C₁₋₅-alkylgruppe (die gleich oder verschieden und an ein oder mehreren Positionen durch ein Halogenatom, eine C₁₋₅-Alkylgruppe, eine C₁₋₅-Alkoxygruppe, eine Nitrogruppe oder eine Cyangruppe substituiert sein kann), eine C₁₋₅-Cycloalkylcarbonylgruppe, eine C₁₋₅-Halogenalkylcarbonylgruppe, eine C₁₋₅-Alkoxy-C₁₋₅-alkylcarbonylgruppe, eine C₁₋₅-Cyanalkylcarbonylgruppe, eine Phenoxy-C₁₋₅-alkylcarbonylgruppe, eine Phenyl-C₁₋₅-alkylcarbonylgruppe, eine Halogenphenyl-C₁₋₅-alkylcarbonylgruppe, eine Benzoylgruppe (die durch eine C₁₋₅--Alkylgruppe, eine C₁₋₅-Halogenalkylgruppe, ein Halogenatom, eine C₁₋₅-Alkoxygruppe, eine Cyangruppe oder eine Nitrogruppe substituiert ist), eine Furylcarbonylgruppe, eine Pyridylcarbonylgruppe, eine Pyrrolylcarbonylgruppe, eine Thienylcarbonylgruppe, eine C₂₋₆-Alkenylcarbonylgruppe, eine Phenyl-C₂₋₆-alkenylcarbonylgruppe, eine Hydroxycarbonyl-C₂₋₆-alkenylcarbonylgruppe, eine C₁₋₅-Alkoxycarbonyl-C₁₋₅-alkylcarbonylgruppe, eine C₁₋₅-Alkoxy-C₁₋₅-alkoxycarbonylgruppe, eine C₁₋₅-Monoalkylaminocarbonylgruppe, eine C₁₋₅-Dialkylaminocarbonylgruppe, eine Phenylaminocarbonylgruppe, eine Halogenbenzylaminocarbonylgruppe, eine C₁₋₅-Alkoxycarbonylgruppe, eine C₁₋₅-Halogenalkoxycarbonylgruppe, eine Benzyloxycarbonylgruppe, eine C₂₋₆-Alkenyloxycarbonylgruppe, eine C₂₋₆-Alkinyloxycarbonylgruppe, eine C₁₋₅-Alkylsulfonylgruppe, eine C₁₋₅-Halogenalkysulfonylgruppe, eine Benzylsulfonylgruppe, die durch eine C₁₋₅-Alkoxycarbonylgruppe substituiert sein kann, eine Phenylsulfonylgruppe, die durch eine C₁₋₅-Alkoxycarbonylgruppe substituiert sein kann, eine Halogenphenylsulfonylgruppe, eine C₁₋₅-Alkylthiocarbonylgruppe, eine C₁₋₅-Halogenalkylthiocarbonylgruppe, eine Benzylthiocarbonylgruppe, eine Halogenbenzylthiocarbonylgruppe, eine C₂₋₆-Alkenylthiocarbonylgruppe, eine C₂₋₆-Alkinylthiocarbonylgruppe, die durch eine Cyangruppe substituiert sein kann, eine C₁₋₅-Alkylamino(thiocarbonyl)gruppe, eine Phenylamino(thiocarbonyl)gruppe, eine C₁₋₅-Dialkylamino(thiocarbonyl)gruppe, eine Benzyloxygruppe oder eine Gruppe der Formel 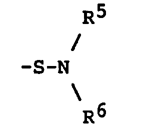 worin jedes von R⁵ und R⁶, die gleich oder verschieden sein können, ein Wasserstoffatom, eine C₁₋₅-Alkylgruppe oder eine C₁₋₅-Alkoxycarbonylgruppe ist, oder R⁵ und R⁶ zusammen mit dem benachbarten Stickstoffatom eine Morpholinogruppe bilden oder R³ und R⁴ zusammen mit dem benachbarten Stickstoffatom eine Azidogruppe, eine Isothiocyanatgruppe, eine Phthalimidgruppe, eine Maleimidgruppe, eine Succinimidogruppe, eine Pyrrolidinylgruppe, eine Piperidinogruppe, eine Pyrrolylgruppe, eine Morpholinogruppe, eine Gruppe der Formel 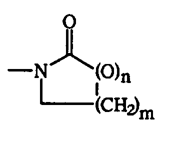 bilden, worin n 0 oder 1 ist und m 1 oder 2 ist oder eine Gruppe der Formel 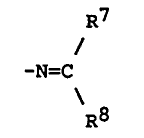 worin jedes von R⁷ und R⁸, die gleich oder verschieden sein können, ein Wasserstoffatom, eine C₁₋₅-Alkylgruppe, eine C₁₋₅-Dialkylaminogruppe, eine Aminogruppe, eine C₁₋₅-Alkylthiogruppe, eine Phenylgruppe oder eine Benzylgruppe ist, R⁹ ein Wasserstoffatom, eine C₁₋₅-Alkylgruppe, eine C₂₋₆-Alkenylgruppe, eine Benzylgruppe, ein Alkalimetallatom, ein Erdalkalimetallatom oder ein orgnisches Aminkation ist oder ein Salz davon.

2. Herbizide Zusammensetzung, umfassend eine herbizid wirksame Menge eines Picolinsäure-Derivats der Formel (I) oder eines Salzes davon, wie in Anspruch 1 definiert, und einen landwirtschaftlichen Zusatz.

3. Picolinsäure-Derivat der Formel 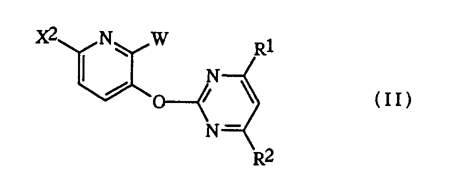 worin jedes von R¹ und R² eine C₁₋₅-Alkoxygruppe, X² eine Aminogruppe, eine C₁₋₅-Alkylaminogruppe oder eine C₁₋₅-Dialkylaminogruppe ist, und W COOR¹⁰, COSR¹¹ oder 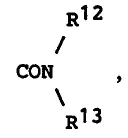 worin R¹⁰ eine Phenylgruppe (die durch ein Halogenatom oder eine Methylgruppe substituiert sein kann), eine Benzyloxy-C₁₋₅-alkylgruppe, eine C₃₋₁₂-Alkylidenaminogruppe, eine C₃₋₈-Cycloalkylidenaminogruppe oder eine C₁₋₅-Dialkylaminogruppe ist, R¹¹ eine C₁₋₅-Alkylgruppe oder eine Phenylgruppe ist, und jedes von R¹² und R¹³, die gleich oder verschieden sein können, ein Wasserstoffatom, eine C₁₋₅-Alkylsulfonylgruppe, eine C₁₋₅-Alkoxy-C₁₋₅-alkylgruppe, eine C₁₋₅-Alkylgruppe, eine Benzyloxygruppe oder eine C₁₋₅-Alkoxygruppe ist, oder R¹² und R¹³ zusammen mit dem benachbarten Stickstoffatom eine Imidazolylgruppe bilden oder ein Salz davon.

4. Herbizide Zusammensetzung, Umfassend eine herbizid wirksame Menge eines Picolinsäure-Derivats der Formel (II) oder eines Salzes davon, wie in Anspruch 3 definiert, und einen landwirtschaftlichen Zusatz.

## Revendications

1. Dérivé d'acide picolinique de formule : 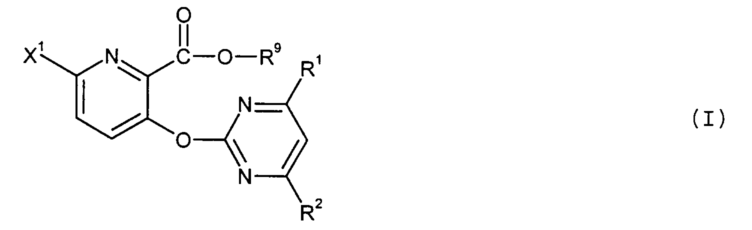 dans laquelle chacun de R¹ et R² est un groupe alcoxy en C₁ à C₅, X¹ est un groupe de formule 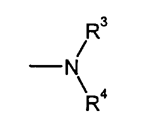 dans laquelle R³ est un atome d'hydrogène, un groupe alkyle en C₁ à C₅, un groupe halogénoalkyle en C₁ à C₅, un groupe (alcoxy en C₁ à C₅)alkyle en C₁ à C₅, un groupe alcényle en C₂ à C₆, un groupe alcynyle en C₂ à C₆, un groupe (alkyl en C₁ à C₅)carbonyle, un groupe benzyloxycarbonyle ou un groupe phénylsulfonyle, et R⁴ est un groupe halogénoalkyle en C₁ à C₅, un groupe (alcoxy en C₁ à C₅)alkyle en C₁ à C₅, un groupe (alcoxy en C₁ à C₅ alkyloxy en C₁ à C₅)alkyle en C₁ à C₅, un groupe (alcoxy en C₁ à C₅)carbonylalkyle en C₁ à C₅, un groupe benzyloxyalkyle en C₁ à C₅, un groupe (alkyl en C₁ à C₅)dioxyalkyle en C₁ à C₅, un groupe phénoxyalkyle en C₁ à C₅, un groupe di (alkyl en C₁ à C₅ aminoalkyle en C₁ à C₅, un groupe cyanoalkyle en C₁ à C₅, un groupe alcoxy en C₁ à C₅, un groupe cycloalkyle en C₃ à C₆, un groupe alcényle en C₂ à C₆ (qui peut être substitué en une ou plusieurs positions par un atome d'halogène ou un groupe cyano), un groupe alcynyle en C₂ à C₆, un groupe phénylalkyle en C₁ à C₅ (qui peut être identique ou différent, et être substitué en une ou plusieurs positions par un atome d'halogène, un groupe alkyle en C₁ à C₅, un groupe alcoxy en C₁ à C₅, un groupe nitro ou un groupe cyano), un groupe (cycloalkyl en C₁ à C₅)carbonyle, un groupe halogénoalkyl en C₁ à C₅)carbonyle, un groupe (alcoxy en C₁ à C₅ alkyl en C₁ à C₅)carbonyle, un groupe (cyanoalkyl en C₁ à C₅)carbonyle, un groupe (phénoxyalkyl en C₁ à C₅)carbonyle, un groupe (phénylalkyl en C₁ à C₅)carbonyle, un groupe (halogénophénylalkyl en C₁ à C₅)carbonyle, un groupe benzoyle (qui est substitué par un groupe alkyle en C₁ à C₅, un groupe halogénoalkyle en C₁ à C₅, un atome d'halogène, un groupe alcoxy en C₁ à C₅, un groupe cyano ou un groupe nitro), un groupe furylcarbonyle, un groupe pyridylcarbonyle, un groupe pyrrolylcarbonyle, un groupe thiénylcarbonyle, un groupe (alcényl en C₂ à C₆)carbonyle, un groupe (phénylalcényl en C₂ à C₆)carbonyle, un groupe (hydroxycarbonylalcényl en C₂ à C₆)carbonyle, un groupe (alcoxy en C₁ à C₅)carbonyl(alkyl en C₁ à C₅)carbonyle, un groupe (alcoxy en C₁ à C₅ alcoxy en C₁ à C₅)carbonyle, un groupe mono(alkyl en C₁ à C₅)aminocarbonyle, un groupe di(alkyl en C₁ à C₅)aminocarbonyle, un groupe phénylaminocarbonyle, un groupe benzylaminocarbonyle, un groupe halogénobenzylaminocarbonyle, un groupe (alcoxy en C₁ à C₅)carbonyle, un groupe (halogénoalcoxy en C₁ à C₅)carbonyle, un groupe benzyloxycarbonyle, un groupe (alcényl en C₂ à C₆)oxycarbonyle, un groupe (alcynyl en C₂ à C₆)oxycarbonyle, un groupe alkylsulfonyle en C₁ à C₅, un groupe halogénoalkylsulfonyle en C₁ à C₅, un groupe benzylsulfonyle qui peut être substitué par un groupe (alcoxy en C₁ à C₅)carbonyle, un groupe phénylsulfonyle qui peut être substitué par un groupe (alcoxy en C₁ à C₅)carbonyle, un groupe halogenophénylsulfonyle, un groupe (alkyl en C₁ à C₅)thiocarbonyle, un groupe (halogénoalkyl en C₁ à C₅)thiocarbonyle, un groupe benzylthiocarbonyle, un groupe halogénobenzylthiocarbonyle, un groupe (alcényl en C₂ à C₆)thiocarbonyle, un groupe (alcynyl en C₂ à C₆)thiocarbonyle qui peut être substitué par un groupe cyano, un groupe (alkyl en C₁ à C₅)aminothiocarbonyle, un groupe phénylaminothiocarbonyle, un groupe di (alkyl en C₁ à C₅)aminothiocarbonyle, un groupe benzyloxy ou un groupe de formule : 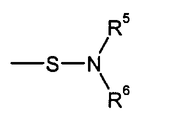 dans laquelle chacun de R⁵ et R⁶, qui peuvent être identiques ou différents, est un atome d'hydrogène, un groupe alkyle en C₁ à C₅ ou un groupe (alcoxy en C₁ à C₅)carbonyle, ou bien R⁵ et R⁶ forment ensemble, conjointement avec l'tome d'azote adjacent, un groupe morpholino,
ou bien R³ et R⁴ forment ensemble, conjointement avec l'atome d'azote adjacent, un groupe azido, un groupe isothiocyanate, un groupe phtalimide, un groupe maléimide, un groupe succinimido, un groupe pyrrolidinyle, un groupe pipéridino, un groupe pyrrolyle, un groupe morpholino, un groupe de formule : 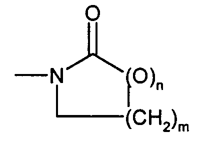 dans laquelle n vaut 0 ou 1, et m vaut 1 ou 2, ou un groupe de formule : 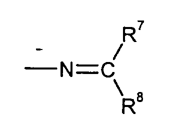 dans laquelle chacun de R⁷ et R⁸, qui peuvent être identiques ou différents, est un atome d'hydrogène, un groupe alkyle en C₁ à C₅, un groupe di(alkyl en C₁ à C₅)amino, un groupe amino, un groupe alkylthio en C₁ à C₅, un groupe phényle ou un groupe benzyle, et R⁹ est un atome d'hydrogène, un groupe alkyle en C₁ à C₅, un groupe alcényle en C₂ à C₆, un groupe benzyle, un atome de métal alcalin, un atome de métal alcalino-terreux ou un cation d'amine organique ;
ou un sel d'un tel dérive.

2. Composition herbicide comprenant une quantité efficace, du point de vue herbicide, d'un dérivé d'acide picolinique de formule (I) ou d'un de ses sels, tel que définis dans la revendication 1, et un adjuvant pour l'agriculture.

3. Dérivé d'acide picolinique de formule : 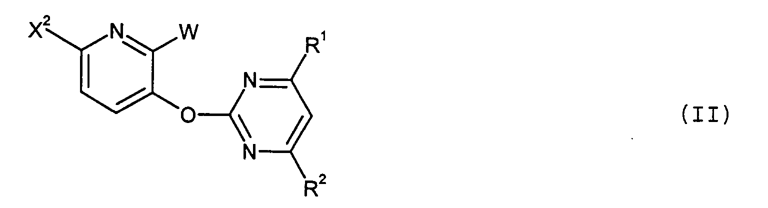 dans laquelle chacun de R¹ et R² est un groupe alcoxy en C₁ à C₅, X² est un groupe amino, un groupe alkylamino en C₁ à C₅ ou un groupe dialkyl en C₁ à C₅)amino, et W est COOR¹⁰, COSR¹¹ ou 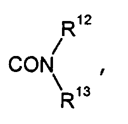 où R¹⁰ est un groupe phényle (qui peut être substitué par un atome d'halogène ou un groupe méthyle), un groupe benzyloxyalkyle en C₁ à C₅, un groupe alkylidène-amino en C₃ à C₁₂, un groupe cycloalkylidèneamino en C₃ à C₈ ou un groupe di(alkyl en C₁ à C₅)amino, R¹¹ est un groupe alkyle en C₁ à C₅ ou un groupe phényle, et chacun de R¹² et R¹³, qui peuvent être identiques ou différents, est un atome d'hydrogène, un groupe alkylsulfonyle en C₁ à C₅, un groupe (alcoxy en C₁ à C₅)alkyle en C₁ à C₅, un groupe alkyle en C₁ à C₅, un groupe benzyloxy ou un groupe alcoxy en C₁ à C₅, ou bien R¹² et R¹³ forment ensemble, conjointement avec l'atome d'azote adjacent, un groupe imidazolyle ; ou un sel d'un tel dérive.

4. Composition herbicide comprenant une quantité efficace, du point de vue herbicide, d'un dérivé d'acide picolinique de formule (II) ou d'un de ses sels, tels que définis dans la revendication 3, et un adjuvant pour l'agriculture.
